(19) Europäisches Patentamt
European Patent Office
Office européen des brevets

(11) Publication number: **0 104 028 B1**

(12) **EUROPEAN PATENT SPECIFICATION**

(45) Date of publication of patent specification: **03.08.88**

(51) Int. Cl.⁴: **C 07 H 17/08**, A 61 K 31/70, A 23 K 1/17

(21) Application number: **83305240.0**

(22) Date of filing: **08.09.83**

# (54) C-20-modified macrolide derivatives.

(30) Priority: **13.09.82 US 417248**

(43) Date of publication of application:
**28.03.84 Bulletin 84/13**

(45) Publication of the grant of the patent:
**03.08.88 Bulletin 88/31**

(84) Designated Contracting States:
**BE CH DE FR GB IT LI NL SE**

(56) References cited:
**EP-A-0 087 921**
**GB-A-2 058 765**

**CHEM. PHARM. BULL., vol. 30, no. 1, 1982, pages 97-110**

(73) Proprietor: **ELI LILLY AND COMPANY**
**307, East McCarty Street**
**Indianapolis Indiana 46285 (US)**

(72) Inventor: **Kirst, Herbert Andrew**
**1819 Madison Village, Apt. B-6**
**Indianapolis Indiana 46227 (US)**
Inventor: **Toth, John Eldon**
**121 West Wood Street, Apt. 5**
**West Lafayette Indiana 47906 (US)**

(74) Representative: **Crowther, Terence Roger et al**
**Erl Wood Manor**
**Windlesham Surrey GU20 6PH (GB)**

**EP 0 104 028 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**Description**

This invention provides a group of compounds which are C-20-modified derivatives of the macrolide antibiotics tylosin, desmycosin, macrocin, lactenocin, 2'''-O-demethylmacrocin (DOMM) and 2''-O-demethyllactenocin (DOML). The compounds are useful as antibiotics or as intermediates to antibiotics.

Certain C-20-modified tylosin derivatives are disclosed in Chem. Pharm. Bull. *30* (1) 1982, pp 97—110 in GB—A—2058765 and in EP—A—0087921, the latter falling under EPC Art. 54(3).

Other C-20-modified tylosin derivatives are claimed in our divisional application EP—A—0203621.

More specifically, the present invention provides macrolides of the formula (I)

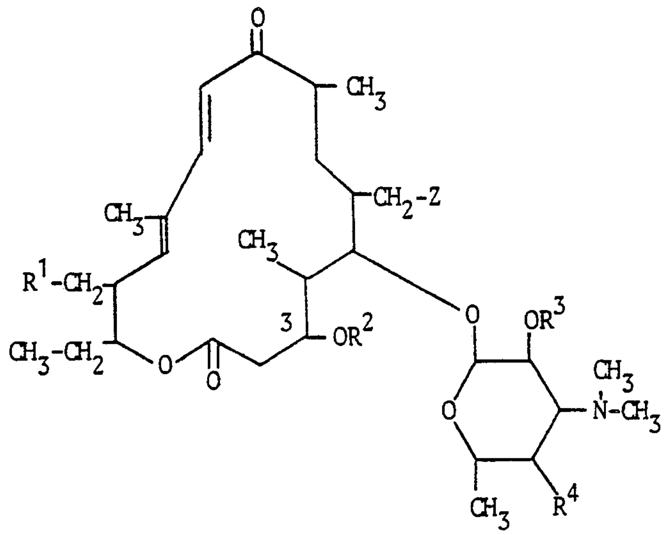

(I)

wherein

$R^1$ is

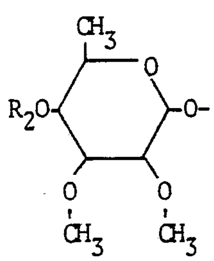 ; 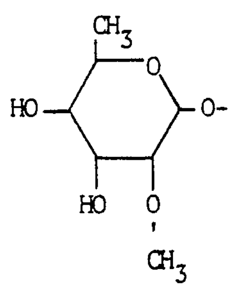 or 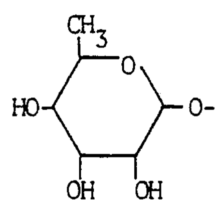 ;

$R^2$ is hydrogen or a hydroxyl protecting group;

$R^3$ is hydrogen, optionally substituted $C_1$—$C_5$-alkanoyl optionally substituted benzoyl, optionally substituted phenylacetyl or optionally substituted phenylpropionyl;

$R^4$ is hydrogen, iodo, hydroxy, optionally substituted $C_1$—$C_5$-alkanoyloxy, optionally substituted benzoyloxy, optionally substituted phenylacetoxy or optionally substituted phenoxyacetoxy or

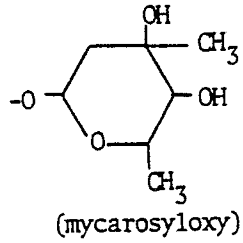

(mycarosyloxy) ;

Z is a group $—CH_2R$, where R is chloro, bromo, azido, or

R is a group —O— $R^{5'}$ where $R^{5'}$ is phenyl, derivatized phenyl, or naphthyl or

R is a group

$$—O—\overset{\overset{\displaystyle O}{\|}}{C}R^6$$

where $R^6$ is optionally substituted $C_1$—$C_4$ alkyl, phenyl, derivatized phenyl, optionally substituted phenoxy, or optionally substituted phenylthio, or

R is a group —$OSO_2R^7$ where $R^7$ is methyl, trifluoromethyl, or optionally substituted phenyl, or

R is a group —$SR^9$ where $R^9$ is optionally substituted $C_1$—$C_4$ alkyl, cyclohexyl, phenyl, derivatized phenyl, optionally substituted benzyl, optionally substituted phenylacetyl, or

an optionally substituted heteroaryl group selected from imidazolyl, pyrazolyl, pyridinyl, pyrimidinyl, pyrazinyl, pyridazinyl, triazinyl, triazolyl, tetrazolyl, oxazolyl, isoxazolyl, oxadiazolyl, thiazolyl, isothiazolyl, thiadiazolyl, thienyl and furanyl, or

R is a group

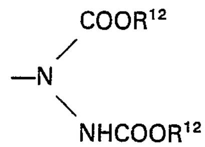

where $R^{12}$ is methyl, or ethyl, or

R is phthalimido or succinimido, or an acid addition salt thereof;

provided that when Z is —$CH_2Br$ then $R^1$ is

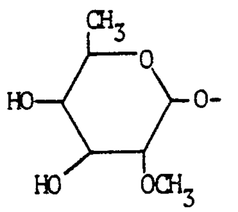

or

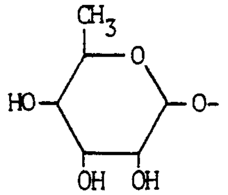

;

and provided that if $R^4$ is hydrogen or iodo, then $R^1$ is

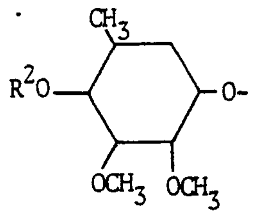

and provided that $R^2$ is hydrogen unless $R^4$ is hydrogen or iodo.

Although no stereochemical assignments are indicated in formula (I), it is to be understood that the stereochemistry is that of tylosin.

The term "$C_1$—$C_5$-alkanoyl" as used herein refers to an acyl moiety derived from a carboxylic acid containing from one to five carbon atoms. In such a moiety, the alkyl group can be straight, branched, or cyclic. When optionally substituted, the alkyl group can bear one to three halo substituents. Halo substituents are selected from the group consisting of Cl, Br and F. Acetyl, chloroacetyl, trichloroacetyl, trifluoroacetyl, propionyl, *n*-butyryl, isobutyryl, *n*-valeryl, and isovaleryl are examples of such groups. The term "$C_1$—$C_5$-alkanoyloxy" refers to the corresponding acyloxy moiety.

The terms "optionally substituted benzoyl, phenylacetyl, phenylpropionyl, phenoxyacetyl or phenylthioacetyl", "optionally substituted benzoyl, phenylacetyl or phenylpropionyl", "optionally substituted benzoyloxy, phenylacetoxy or phenoxyacetoxy", "optionally substituted phenyl, benzyl or phenethyl", "optionally substituted benzyl, phenethyl or phenoxyethyl" and "optionally substituted phenyl" means that the phenyl portion of the moiety is optionally substituted by from one to five halo or methyl groups or by from one to two methoxyl, nitro or hydroxyl groups.

The term "derivatized phenyl" refers to a phenyl group which has from one to five halo, methoxyl or $C_1$—$C_4$-alkyl substituents, or from one to two nitro, amino, methylamino, ethylamino, dimethylamino, diethylamino, $C_4$—$C_{10}$-methyleneamino, azido, hydroxy, hydroxymethyl, aminomethyl, (methylamino)-methyl, (ethylamino)methyl, (dimethylamino)methyl, (diethylamino)methyl, ($C_4$—$C_7$-methyleneamino)-methyl, formyl, acetyl, benzoyl, methoxycarbonyl, methyl, (ethylamino) methyl, (dimethylamino) ethoxycarbonyl, carboxamido, N-methylcarboxamido, N,N-dimethylcarboxamido, cyano, phenyl, phenoxy or benzyl substituents.

The term "optionally substituted heteroaryl group" as used herein means that the heteroaryl group may have at least one suitable substituent(s) such as a $C_1$—$C_4$-alkyl, halo, methoxy, ethoxy, hydroxy (or the keto tautomer) or phenyl group.

The term "$C_1$—$C_4$-alkyl" as used herein means a straight- or branched-chain alkyl group containing from one to four carbon atoms. Such groups include methyl, ethyl, isopropyl, *n*-butyl, *tert*-butyl, and the like. "Optionally substituted $C_1$—$C_4$-alkyl" refers to a $C_1$—$C_4$-alkyl group which contains one or more fluoro, chloro, bromo or iodo substituents on the alkyl group.

The $R^{11}$ "alkoxycarbonyl" term represents a member of a group selected from t-butoxycarbonyl, methoxycarbonyl, ethoxycarbonyl, 2,2,2-trichloroethoxycarbonyl, phenoxycarbonyl and benzyloxy-carbonyl.

The term "$C_4$—$C_{10}$-methyleneamino" represents a cyclic amino substituent of the formula $—N(CH_2)_n$ wherein n is an integer from four to ten. Pyrrolidinyl, piperidinyl, and octahydroazocinyl are examples of such groups.

The term "hydroxyl-protecting group" refers to a substituent which is not removed under the reaction conditions but which can be readily removed after the reaction has been completed to liberate the original hydroxyl group. Hydroxyl-protecting groups are well known in the art (see, for example, T. W. Greene, Protective Groups in Organic Synthesis", Wiley-Interscience, 1981, pp. 10—86). One especially suitable hydroxyl-protecting group is the tetrahydropyranyl group.

Preparing Macrolides of Formula (I)

Macrolides of formula (I) are prepared from corresponding macrolides of formula (II)

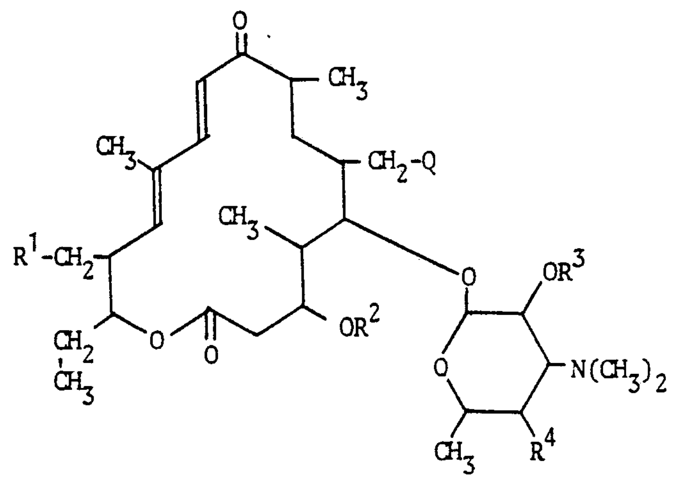

(II)

where $R^1$, $R^2$, $R^3$, and $R^4$ are as previously defined in formula (I) and Q is $CH_2OH$, CHO, $—CH_2L$, $—CH_2N_3$, or $—CH_2NH_2$, where L represents a leaving group. Good leaving groups include sulfonic acid ester groups such as methanesulfonyloxy, trifluoromethanesulfonyloxy, and optionally substituted phenylsulfonyloxy, and halo, including iodo and bromo.

Aldehyde starting materials, i.e., macrolides of formula (II) wherein Q is —CHO include the following known macrolides of formula (II) and esters thereof:

| | $R^2$ | $R^3$ | $R^4$ | $R^1$ |
|---|---|---|---|---|
| tylosin | H | H | mycarosyloxy | mycinosyloxy |
| desmycosin | H | H | OH | mycinosyloxy |
| 4′-deoxydesmycosin | H | H | H | mycinosyloxy |
| macrocin | H | H | mycarosyloxy | 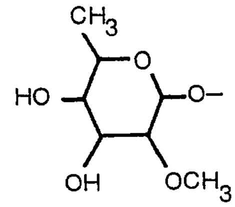 |
| lactenocin | H | H | OH | 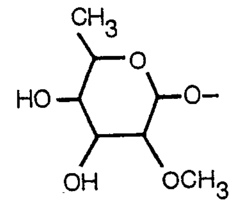 |
| 2‴-O-demethylmacrocin (DOMM) | H | H | mycarosyloxy | 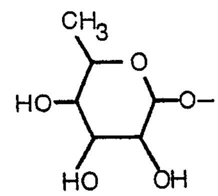 |
| 2‴-O-demethylacetenocin (DOML) | H | H | OH | 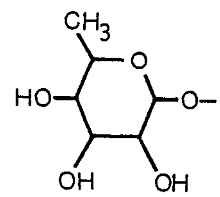 |

Tylosin and its preparation by fermentation of *Streptomyces fradiae* NRRL 2702 or 2703 are described in U.S. Patent No. 3,178,341. Desmycosin and preparation of desmycosin by mild acid hydrolysis of tylosin are also described in U.S. Patent No. 3,178,341.

4′-Deoxydesmycosin and a method of preparing it from desmycosin are described in A. Tanaka et al., *J. Antibiotics 34,* 1381—84 (1981).

Macrocin and its preparation by fermentation of *Streptomyces fradiae* NRRL 2702 or 2703 are described in U.S. Patent No. 3,326,759. Lactenocin and preparation of lactenocin by mild acid hydrolysis of macrocin are also described in U.S. Patent No. 3,326,759.

DOMM and its preparation by fermentation of *Streptomyces fradiae* ATCC 31669 are described in EPO published specification 45157. DOML and preparation of DOML by mild acid hydrolysis of DOMM are also described in EPO published specification 45157.

Aldehyde starting materials of formula (II) which are 2′- or 4′-monoesters, or 2′,4′-diesters of the above described known macrolides, i.e., macrolides of formula (II) wherein $R^3$ is other than hydrogen or $R^4$ is other than hydroxyl, or both, are prepared by known acylation procedures. Typical acylating agents include activated carboxylic acid derivatives such as anhydrides, acid halides (usually in combination with a base or other acid scavenger) and active esters. Suitable organic solvents for this reaction include pyridine and triethylamine. Acylation can also be achieved using a mixture of an organic acid and a dehydrating agent such as N,N′-dicyclohexylcarbodiimide. Once formed, the acyl derivative can be separated and purified by known techniques.

Aldehyde starting materials of Formula (II) which are 2′-monoester derivatives can be prepared by selective esterification of compounds of formula (II) wherein $R^3$ is hydrogen and $R^4$ is hydroxyl using the technique described in U.S. Patents 4,321,361 and 4,321,362. 2′-Hydroxyl groups are more facile to esterification than 4′-hydroxyl groups. Accordingly, the 2′-monoester derivative can be selectively prepared by, for example, treating the macrolide starting material with a stoichiometric quantity (or a slight excess) of an acylating agent, such as an acyl anhydride, at about room temperature for from about 1 to about 24 hours until esterification is substantially complete. The 2′-monoester can be isolated from the

reaction mixture by standard procedures such as extraction, chromatography and crystallization.

Aldehyde starting materials which are 2′,4′-diester derivatives are prepared from macrolides of formula (II) wherein $R^3$ is hydrogen and $R^4$ is hydroxyl using the procedure described in published European Patent specification 82,003. thus, symmetrical 2′,4′-diester derivatives are prepared by treating the known macrolide of formula (II) wherein $R^3$ is hydrogen and $R^4$ is hydroxyl with a stoichiometric quantity (or a slight excess) of an acylating agent, such as an acyl anhydride, in a neutral solvent such as acetone, at about room temperature for from 1 to about 24 hours until esterification of the 2′ and 4′ hydroxyl groups is complete. Unsymmetrical 2′,4′-diester derivatives, i.e., compounds of formula (II) wherein $OR^3$ and $R^4$ are different, can be prepared by acylation of appropriate 2′-monoesters.

Starting materials of formula (II) wherein Q is —$CH_2OH$ include the following macrolides, and esters thereof:

| | $R^2$ | $R^3$ | $R^4$ | $R^1$ |
|---|---|---|---|---|
| dihydrotylosin (relomycin) | H | H | mycarosyloxy | mycinosyloxy |
| dihydrodesmycosin | H | H | OH | mycinosyloxy |
| dihydromacrocin | H | H | mycarosyloxy | 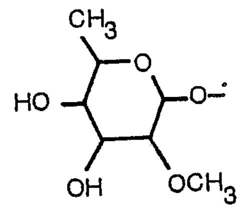 |
| dihydrolactenocin | H | H | OH | 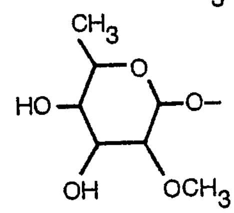 |
| dihydro-DOMM | H | H | mycarosyloxy | 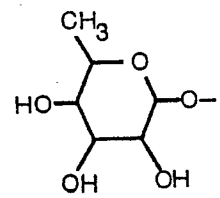 |
| dihydro-DOML | H | H | OH | 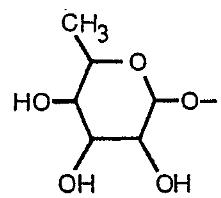 |

These macrolides are prepared by reducing the aldehyde group of tylosin, desmycosin, macrocin, lactenocin, DOMM, and DOML, or esters thereof.

Starting materials of formula (II) wherein Q is $CH_2L$ are prepared by converting the C-20 hydroxyl group of a macrolide of formula (II) wherein Q is —$CH_2OH$ macrolides to the desired leaving group L by methods known per se. For example, the C-20 hydroxyl group can be converted to the triflate group by reacting the macrolide with an activated derivative of trifluoromethane sulfonic acid such as trifluoromethane sulfonic anhydride or trifluoromethane sulfonylchloride in the presence of a base in a non-reactive organic solvent. If desired, the triflate group may be converted to the iodo group, for example by reacting the unisolated sulfonic ester intermediate with a source of iodide ion such as tetra n-butylammonium iodide, or sodium iodide. In case of dihydrodesmycosin, dihydrolactenocin, and dihydro-DOML, the 20-iodo derivative can be formed directly by adding iodine dissolved in a suitable solvent, such as dimethylformamide, to a solution of the 20-dihydro macrolide derivative and triphenylphosphine under nitrogen.

Starting materials of formula (II) wherein Q is —$CH_2N_3$ are novel compounds, the preparation of which will be described hereinafter. Starting materials of formula (II) wherein Q is —$CH_2NH_2$ can be prepared by reacting a macrolide of formula (II) wherein Q is —$CH_2N_3$ with a reducing agent such as triphenylphosphine.

Macrolides of formula (I) wherein Z is —$CH_2Cl$, or —$CH_2Br$ are prepared by reacting a macrolide of formula (II) where Q is —$CH_2OH$ with triphenylphosphine and a halogenating agent in a non-reactive

organic solvent such as dichloromethane. Suitable halogenating agents include alkyl halides and polyhalides, such as carbon tetrachloride. This method is illustrated, for example, in Example 5.

Macrolides of formula (I) wherein Z is $—CH_2Cl$, or $—CH_2Br$ are also prepared by reacting a macrolide of formula (II) wherein Q is $—CH_2OH$ with triphenylphosphine, diethylazodicarboxylate or dimethylazodicarboxylate, and an alkyl halide or polyhalide.

Macrolides of formula (I) wherein Z is $—CH_2Cl$, or $—CH_2Br$ are also prepared by reacting a macrolide of formula (II) wherein Q is —CHO with an alkali metal or tetra(alkyl)ammonium halide in a nonreactive organic solvent such as tetrahydrofuran. This method is illustrated in Example 23.

Macrolides of formula (I) wherein Z is $—CH_2N_3$ are prepared by reacting a macrolide of formula (II) wherein Q is —CHO with an alkali metal or tetra(alkyl)ammonium azide in a nonreactive organic solvent. Macrolides of formula (I) wherein Z is $—CH_2N_3$ are also prepared by reacting a macrolide of formula (II) wherein Q is $—CH_2OH$ with triphenylphosphine, diethylazodicarboxylate or dimethylazodicarboxylate, and an azide transfer agent, such as diphenylphosphoryl azide, in a nonreactive organic solvent such as tetrahydrofuran. An example of this method is given in Example 11.

Macrolides of formula (I) wherein Z is $CH_2OR^{5'}$ are prepared by reacting a macrolide of formula (II) wherein Q is $—CH_2OH$, with triphenylphosphine, di(alkyl)azodicarboxylate, where alkyl is methyl or ethyl, and a phenol of formula $HOR^{5'}$ where $R^{5'}$ is as defined in formula (I). Use of the di(alkyl)azodicarboxylate/triphenylphosphine reaction in this context is illustrated in Example 7 and 25, for example.

The di(alkyl)azodicarboxylate/triphenylphosphine reaction and its various applications are described in O. Mitsunobu, *Synthesis, 1* (1), 1—28 (1981). The reaction generally produces dehydration between an alcohol ROH and an acidic component HX to provide a product RX.

Macrolides of formula (I) wherein Z is $R^6COOCH_2—$ are prepared by reacting a macrolide of formula (II) wherein Q is $—CH_2OH$ with an acylating agent derived from a carboxylic acid of formula $R^6COOH$, where $R^6$ is as defined in formula (I). Typical acylating agents include anhydrides, acid halides (usually in combination with a base or other acid scavenger), and active esters. Suitable organic solvents include pyridine and triethylamine. Acylation can also be achieved using a mixture of an organic acid and a dehydrating agent such as N,N'-dicyclohexylcarbodiimide. Acylation is illustrated in Examples 9 for example.

Macrolides of formula (I) wherein Z is $R^6COOCH_2—$ are also prepared using the di(alkyl)azodicarboxylate/triphenylphosphine procedure, i.e., by reacting a macrolide of formula (II) wherein Q is $—CH_2OH$ with triphenylphosphine, di(alkyl)azodicarboxylate, and a carboxylic acid of the formula $R^6COOH$, wherein $R^6$ is as defined in formula (I).

Macrolides of formula (I) wherein Z is $R^7SO_2OCH_2—$ are prepared by reacting a macrolide of formula (I) wherein Q is $—CH_2OH$ with an activated derivative, such as the anhydride or acid halide, of a sulfonic acid of the formula $R^7SO_2OH$. If the acid halide is used, the reaction is carried out in the presence of a base, usually pyridine.

Macrolides of formula (I) wherein Z is $R^7SO_2OCH_2—$ are also prepared using the di(alkyl)azodicarboxylate/triphenylphosphine procedure, i.e., by reacting a macrolide of formula (II) wherein Q is $—CH_2OH$ with triphenylphosphine, di(alkyl)azodicarboxylate and a sulfonic acid of the formula $R^7SO_2OH$, where $R^7$ is as defined in formula (I).

Macrolides of formula (I) wherein Z is $—CH_2SR^9$ are prepared by reacting a macrolide of formula (II) wherein Q is $—CH_2L$ with a mercaptide ion of the formula $R^9S^-$. L may be halide or a sulfonic or sulfonic ester group.

Macrolides of formula (I) wherein Z is $—CH_2SR^9$ are also prepared using the di(alkyl)azodicarboxylate/triphenylphosphine procedure, i.e., by reacting a macrolide of formula (II) wherein Q is $—CH_2OH$ with triphenylphosphine, di(alkyl)azodicarboxylate and a mercaptan of formula $HSR^9$. This method is illustrated in Example 3.

Macrolides of formula (I) wherein Z is

$$—CH_2N\begin{array}{l} \diagup COOR^{12} \\ \diagdown NHCOOR^{12} \end{array}$$

where $R^{12}$ is methyl or ethyl are prepared by reacting a macrolide of formula (II) wherein Q is $—CH_2OH$ with triphenylphosphine and diethylazodicarboxylate or dimethylazodicarboxylate.

Macrolides of formula (I) wherein Z is $—CH_2$-phthalimido or $—CH_2$-succinimido are prepared by reacting a macrolide of formula (II) wherein Q is $—CH_2OH$ with triphenylphosphine, di(alkyl)azodicarboxylate, and phthalimide or succinimide.

When a product of formula (I) is one in which $R^4$ is mycarosyloxy, the corresponding macrolide of formula (I) wherein $R^4$ is hydroxyl can be prepared by acid hydrolysis of the initial product. More specifically, the mycarose sugar can be hydrolytically cleaved at a pH of less than 4, preferably in the range from 0.5 to 2.0, at a temperature in the range of from 0 to 60°C, conveniently at about room temperature. The hydrolysis can be effected using a strong aqueous mineral acid such as hydrochloric or sulfuric acid or a strong organic acid such as *p*-toluenesulfonic acid.

As previously mentioned, a method of preparing 4'-deoxydesmyosin is described in *J. of Antibiotics 34,* 1381—84 (1981). The process involves (1) treatment of desmyosin with acidic ethanol in accordance with a procedure described in *Antibiot. & Chemoth. 11,* 320—27 (1961), to obtain the corresponding diethylacetal; (2) acylation of the diethylacetal with acetic anhydride in acetonitrile in the absence of external base, in accordance with a procedure described in *J. Org. Chem. 44,* 2050—52 (1979), to obtain the 2',4'-di-O-acetyl derivative; (3) reacting the 2',4'-di-O-acetyl derivative with 2,3-dihydrofuran in dichloromethane in the presence of pyridinium p-toluenesulfonate in the manner described in *J. Org. Chem. 42,* 3772—74 (1974) to obtain the 3,4''-bis(O-tetrahydrofuranyl)derivative; (4) removal of the 2' and 4'-O-acetyl groups by dissolving the product of step (3) in methanol (50°C, overnight); (5) reacting the product of step (4) with 1.5 mole equivalent of benzenesulfonyl chloride in pyridine at −40°C for 4 hours, to provide the 4'-O-benzenesulfonyl derivative; (6) immediately reacting the 4'-O-benzenesulfonyl derivative with 1.5 equivalent of sodium iodide in methyl ethyl ketone at 180°C for 15 minutes to obtain 4' iodo derivative; (7) reductively deiodinating the 4'-iodo derivative using tri(n-butyl)stannane in benzene in the presence of 2,2'-azobisisobutyronitrile at 80°C for 2 hours; and (8) deblocking the diethylacetal and tetrahydrofuranyl groups by hydrolysis of the product of step (7) in .1M aqueous hydrochloric acid-acetonitrile (2.5:1 v/v) for 30 minutes at 25°C to obtain 4'-deoxydesmycosin.

The 4'-deoxydesmycosin thus prepared may then be modified at the C—20, and optionally at the 2' position as described above.

Alternatively, a C—20 modified derivative of 4'-deoxydemycosin may be prepared by deoxygenating a C—20 modified derivative of desmycosin, for example by treating the C—20 modified derivative in accordance with steps 2 through 6 to 2 through 8 of the process of *J. Antibiotics 34,* 1381—84 (1981) as described above.

The procedures used to prepare 2' and 4' esters of the starting macrolides of formula (II) were described above. Macrolides of formula (I) can be acylated using identical procedures to obtain 2'- and 4'-monoesters and 2',4'-diesters of formula (I).

The C—20-modified derivatives of this invention form salts, particularly acid addition salts. These acid addition salts are also useful as antibiotics and are a part of this invention. In another aspect, such salts are useful as intermediates, for example, for separating and purifying the derivatives. In addition, the salts have an improved solubility in water.

Representative suitable salts include those salts formed by standard reactions with both organic and inorganic acids such as, for example, sulfuric, hydrochloric, phosphoric, acetic, succinic, citric, lactic, maleic, fumaric, palmitic, cholic, pamoic, mucic, D-glutamic, *d*-camphoric, glutaric, glycolic, phthalic, tartaric, formic, lauric, stearic, salicyclic, methanesulfonic, benzenesulfonic, sorbic, picric, benzoic, cinnamic, and like acids.

Accordingly, the present invention provides a process for preparing a macrolide of formula (I) or a pharmaceutically-acceptable salt thereof, which comprises reacting a starting macrolide of formula (II)

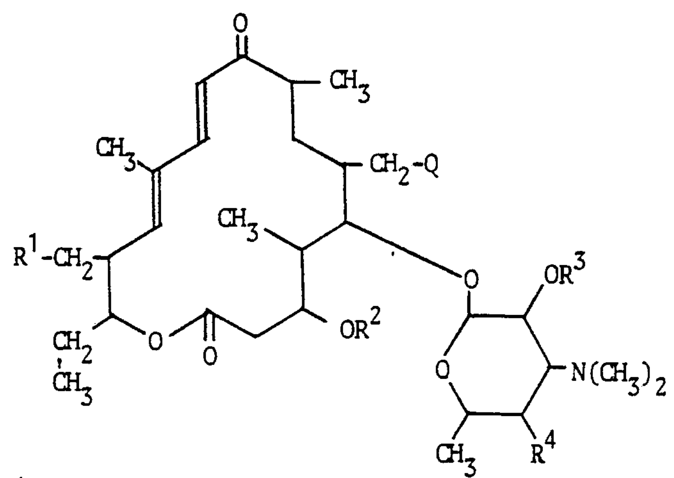

(II)

wherein $R^1$, $R^2$, $R^3$, and $R^4$ are as defined in formula (I), and

(A) where Q is $-CH_2OH$,

with an acylating agent derived from a carboxylic acid of the formula $R^6COOH$ to provide a macrolide of formula (I) wherein Z is $R^6COOCH_2-$, where $R^6$ is as defined in Formula (I), or

with an acylating agent derived from a sulfonic acid of the formula $R^7SO_2OH$ where $R^7$ is as defined in formula (I) to provide a macrolide of formula (I) wherein Z is $R^7SO_2OCH_2-$, or

with triphenylphosphine and a halogenating agent to provide a macrolide of formula (I) wherein Z is $-CH_2Cl$, or $-CH_2Br$, or

with triphenyl phosphine and diethylazodicarboxylate or dimethylazodicarboxylate to provide a macrolide of formula (I) where Z is

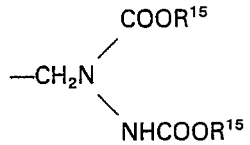

and $R^{15}$ is methyl or ethyl, or

with triphenylphosphine, and diethylazodiocarboxylate or dimethylazodicarboxylate, and a reagent selected from

(a) an azide transfer reagent to provide a macrolide of formula (I) wherein Z is $—CH_2N_3$,

(b) phthalimide or succinimide to provide macrolide of formula (I) wherein Z is $—CH_2—$ phthalimido or $—CH_2$-succinimido,

(c) a phenol of formula $HOR^5$, where $R^5$ is phenyl or derivatized phenyl, to provide a macrolide of formula (I) wherein Z is $—CH_2OR^5$,

(d) a carboxylic acid of formula $R^6COOH$, where $R^6$ is as defined in formula (I), to provide a macrolide of formula (I) wherein Z is $R^6COOCH_2—$,

(e) an alkyl halide or polyhalide to provide a macrolide of formula (I) wherein Z is $—CH_2Br$, or $—CH_2Cl$,

(f) a sulfonic acid of the formula $R^7SO_2OH$, where $R^7$ is as defined in formula (I) to provide a macrolide of formula (I) wherein Z is $—CH_2OSO_2R^7$,

(g) a mercaptan of the formula $HSR^9$, where $R^9$ is as defined in formula (I) to provide a macrolide of formula (I) wherein Z is $—CH_2SR^9$.

B) where Q is $—CH_2L$ and L is a leaving group,

with an alkali metal azide or halide or tetra(alkyl)ammonium azide or fluoride, where alkyl is methyl, ethyl, propyl, or butyl to provide a macrolide of formula (I) wherein Z is $—CH_2N_3$, $—CH_2Cl$, or $—CH_2Br$, or

with a salt of a mercaptan of formula $R^9S—$, where $R^9$ is as defined in formula (I) to provide a macrolide of formula (I) wherein Z is $—CH_2SR^9$, or

or hydrolyzing a macrolide of formula (I) wherein $R^4$ is mycarosyloxy in acid solution at a pH below 4 to provide a corresponding macrolide of formula (I) wherein $R^4$ is hydroxy;

or reductively deiodinating a macrolide of formula I wherein $R^4$ is iodo to provide a macrolide of formula (I) wherein $R^4$ is hydrogen; and

optionally esterifying or salifying the macrolide provided by any of the foregoing steps.

Illustrative C—20-modified derivatives of this invention are listed in Tables I—VII.

TABLE I

Illustrative C—20 Modified Derivatives of Tylosin[a]

| Compound No. | R of Formula (I) |
|---|---|
| 1 | N-phthalimido |
| 2 | azido |
| 4 | phenoxy |
| 5 | (1-methyltetrazol-5-yl)thio |
| 6 | phenylthio |
| 7 | chloro |
| 9 | N-(ethoxycarbonyl),N-(ethoxycarbonylamino)-amino |
| 11 | phenoxyacetoxy |
| 12 | p-nitrophenoxy |
| 13 | p-benzoylphenoxy |
| 14 | p-methoxyphenoxy |
| 15 | 3,5-dichlorophenoxy |
| 16 | m-(N,N-dimethylamino)phenoxy |
| 17 | p-formylphenoxy |
| 18 | p-phenylphenoxy |

[a] $R^1$ = 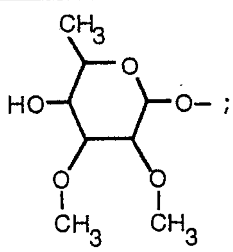 ; $R^3$ = H; and $R^4$ = mycarosyloxy

TABLE II

Illustrative C—20 Modified
Derivatives of Desmycosin[a]

| Compound No. | R of Formula (I) |
|---|---|
| 20 | trifluoromethanesulfonyloxy |
| 21 | chloro |
| 23 | phenylthio |
| 24 | (1-methyltetrazol-5-yl)thio |
| 26 | phenoxyacetoxy |
| 29 | acetoxy |
| 33 | phenoxy |
| 34 | p-nitrophenoxy |
| 35 | p-methoxyphenoxy |
| 36 | p-formylphenoxy |
| 37 | p-benzoylphenoxy |
| 38 | p-(ethoxycarbonyl)phenoxy |
| 39 | m-(N,N-dimethylamino)phenoxy |
| 40 | 3,5-dichlorophenoxy |
| 41 | p-phenylphenoxy |
| 42 | p-(hexahydroazepin-1-ylmethyl)phenoxy |
| 43 | N-phthalimido |
| 44 | azido |
| 45 | N-(ethoxycarbonyl), N-(ethoxycarbonylamino)amino |
| 47 | (p-phenoxy)phenoxy |
| 48 | (m-phenoxy)phenoxy |
| 51a | p-(hydroxymethyl)phenoxy |

[a] $R^1$ = 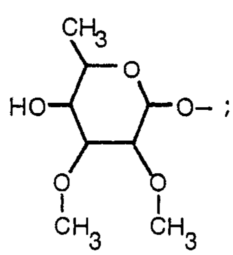 ; $R^3$ = H; $R^4$ = OH

TABLE III

Illustrative C—20 Modified Derivatives of Macrocin[a]

| Compound No. | Z of Formula (I) | R of Formula (I) |
|---|---|---|
| 53 | | 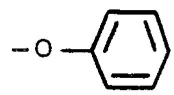 |

[a] $R^1$ = 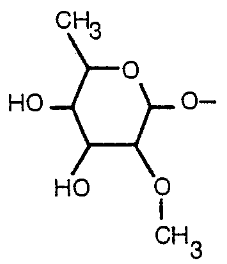 ; $R^3$ = H; and $R^4$ = mycarosyloxy

TABLE IV

Illustrative C—20 Modified Derivatives of Lactenocin[a]

| Compound No. | Z of Formula (I) | R of Formula (I) |
|---|---|---|
| 58 | | 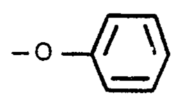 |

[a] R = 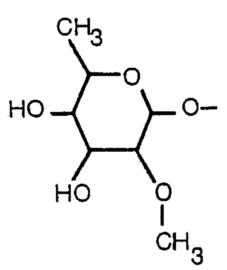 ; $R^3$ = H; and $R^4$ = OH

TABLE V

Illustrative C—20-Modified Ester Derivatives of Tylosin[a]

| Compound No. | R | $R^3$ |
|---|---|---|
| 62 | N-phthalimido | propionyl |
| 63 | chloro | propionyl |
| 64 | phenoxy | acetyl |

[a] $R^1$ = 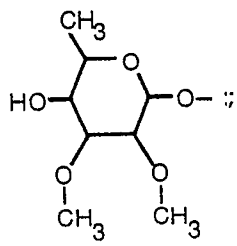 ; $R^4$ = mycarosyloxy

TABLE VI

Illustrative C—20-Modified Ester Derivatives of Desmycosin[a]

| Compound No. | R | $R^3$ | $R^4$ |
|---|---|---|---|
| 65 | N-phthalimido | propionyl | —OH |
| 66 | N-phthalimido | propionyl | —O-propionyl |
| 67 | phenoxy | acetyl | —OH |
| 68 | phenoxy | acetyl | —O-acetyl |
| 69 | chloro | propionyl | —OH |

[a] $R^1$ = 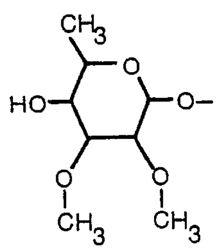

TABLE VII
Illustrative C—20-Modified Derivatives of 4'-Deoxydesmycosin[a]

| Compound No. | R |
|---|---|
| 70 | phenoxy |
| 71 | chloro |
| 72 | N-phthalimido |

[a] $Q^1$ = 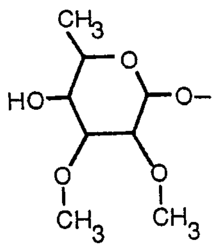 ; $R^2$, $R^3$ and $R^4$ = H.

The derivatives of this invention inhibit the growth of pathogenic bacteria, especially gram-positive bacteria, *Mycoplasma* species and *Pasteurella* species. The derivatives are particularly useful against the *Pasteurella* species *P. multocida* and *P. hemolytica* and against *Mycoplasma hyopneumoniae*, the causative agent of mycoplasmal pneumonia in swine, and *Mycoplasma gallisepticum*.

In addition, many of the C—20-modified compounds of this invention exhibit higher blood levels than those of the parent compounds. For example, after oral administration of a single 15 mg/kg dose to female mongrel dogs, 20-DH-20-O-phenyl-desmycosin (Compound *33*) gave a peak plasma level of 2.1 mcg/ml; whereas erythromycin gave 1.6 mcg/ml and desmycosin only gave 0.5 mcg/ml in analogous experiments.

The minimal inhibitory concentration (MIC's) at which illustrative compounds inhibit certain bacteria are given in Tables IX and X. The MIC's in Table IX were determined by standard agar-dilution assays. The MIC's in Table X were obtained using conventional broth-dilution microtiter tests.

TABLE IX

Antibiotic Activity of C—20 Modified Derivatives[a]

| Test Organism | Test Compound[b] 1 | 2 | 4 | 5 | 6 | 7 | 9 |
|---|---|---|---|---|---|---|---|
| *Staphylococcus aureus* X1.1 | 1 | 2 | 1 | 0.5 | 1 | 1 | 2 |
| *Staphylococcus aureus* V41[c] | 1 | 1 | 1 | 1 | 1 | 1 | 2 |
| *Staphylococcus aureus* X400[d] | 2 | 2 | 2 | 2 | 2 | 2 | 8 |
| *Staphylococcus aureus* S13E | 1 | 1 | 1 | 2 | 1 | 1 | 2 |
| *Staphylococcus epidermidis* EP11 | 1 | 1 | 1 | 1 | 2 | 1 | 4 |
| *Staphylococcus epidermidis* EP12 | 1 | NT | NT | NT | 1 | NT | 2 |
| *Streptococcus pyogenes* C203 | 2 | 2 | 2 | 1 | 4 | 2 | 16 |
| *Streptococcus pneumoniae* Park I | 32 | 32 | 32 | 32 | 64 | 32 | 2 |
| *Streptococcus* Group D X66 | NT[g] | 128 | 128 | 128 | — | — | — |
| *Streptococcus* Group 9960 | 128 | 128 | 128 | 128 | — | — | — |
| *Haemophilus influenzae* Holt[e] | —[h] | — | — | — | — | — | — |
| *Haemophilus influenzae* R252[f] | — | — | — | 128 | — | — | — |

[a] MIC in mcg/ml
[b] Compound numbers from Tables I—VIII
[c] Penicillin-resistant strain
[d] Methicillin-resistant strain
[e] Ampicillin-sensitive strain
[f] Ampicillin-resistant strain
[g] Not tested
[h] Not active at 128 mcg/ml, the highest level tested

TABLE IX cont'd.
Antibiotic Activity of C—20 Modified Derivatives[a]

| Test Organism | Test Compound[b] 11 | 12 | 13 | 14 | 15 | 16 | 17 | 18 |
|---|---|---|---|---|---|---|---|---|
| *Staphylococcus aureus* X1.1 | 0.5 | 1 | 1 | 0.5 | 1 | 1 | 1 | 2 |
| *Staphylococcus aureus* V41[c] | 0.5 | 1 | 1 | 0.5 | 2 | 1 | 1 | 2 |
| *Staphylococcus aureus* X400[d] | 1 | 2 | 4 | 2 | 4 | 2 | 2 | 2 |
| *Staphylococcus aureus* S13E | 0.5 | 1 | 1 | 1 | 1 | 1 | 1 | 2 |
| *Staphylococcus epidermidis* EP11 | 0.5 | 1 | 2 | 2 | 2 | 1 | 1 | 2 |
| *Staphylococcus epidermidis* EP12 | 0.5 | 0.5 | 1 | 1 | 1 | 1 | 0.5 | 0.5 |
| *Streptococcus pyogenes* C203 | 0.5 | 2 | 2 | 1 | 1 | 1 | 1 | 2 |
| *Streptococcus pneumoniae* Park I | 0.5 | 16 | 1 | 8 | 16 | 8 | 16 | 1 |
| *Streptococcus* Group D X66 | 8 | 128 | 4 | 128 | — | 64 | 128 | 8 |
| *Streptococcus* Group 9960 | 8 | —[h] | 4 | 128 | — | — | — | — |
| *Haemophilus influenzae* Holt[e] | — | — | — | 128 | — | 128 | — | — |
| *Haemophilus influenzae* R252[f] | — | 128 | — | — | — | — | — | — |

[a] MIC in mcg/ml
[b] Compound numbers from Tables I—VIII
[c] Penicillin-resistant strain
[d] Methicillin-resistant strain
[e] Ampicillin-sensitive strain
[f] Ampicillin-resistant strain
[g] Not tested
[h] Not active at 128 mcg/ml, the highest level tested

TABLE IX cont'd.
Antibiotic Activity of C—20 Modified Derivatives[a]

| Test Organism | Test Compound[b] 21 | 23 | 24 | 26 | 29 |
|---|---|---|---|---|---|
| *Staphylococcus aureus* X1.1 | 0.25 | 0.25 | 0.5 | 0.25 | 0.5 |
| *Staphylococcus aureus* V41[c] | 0.12 | 0.25 | 0.5 | 0.25 | 0.5 |
| *Staphylococcus aureus* X400[d] | 0.5 | 0.25 | 1 | 0.5 | 0.5 |
| *Staphylococcus aureus* S13E | 0.12 | 0.25 | 0.5 | 0.25 | 0.25 |
| *Staphylococcus epidermidis* EP11 | 0.12 | 0.25 | 0.25 | 1 | 0.25 |
| *Staphylococcus epidermidis* EP12 | NT[g] | 0.12 | NT | 0.12 | 0.12 |
| *Streptococcus pyogenes* C203 | 0.25 | 0.25 | 0.25 | 0.12 | 2 |
| *Streptococcus pneumoniae* Park I | 1 | 2 | 2 | 0.12 | 0.25 |
| *Streptococcus* Group D X66 | 8 | 8 | 16 | 0.5 | 8 |
| *Streptococcus* Group 9960 | 8 | 16 | 32 | 0.5 | NT |
| *Haemophilus influenzae* Holt[e] | 16 | 32 | 32 | 8 | 16 |
| *Haemophilus influenzae* R252[f] | 16 | 32 | 32 | 8 | 16 |

[a] MIC in mcg/ml
[b] Compound numbers from Tables I—VIII
[c] Penicillin-resistant strain
[d] Methicillin-resistant strain
[e] Ampicillin-sensitive strain
[f] Ampicillin-resistant strain
[g] Not tested
[h] Not active at 128 mcg/ml, the highest level tested

TABLE IX cont'd.
Antibiotic Activity of C—20 Modified Derivatives[a]

| Test Organism | Test Compound[b] 33 | 34 | 35 | 36 | 37 | 38 | 39 | 40 |
|---|---|---|---|---|---|---|---|---|
| *Staphylococcus aureus* X1.1 | 0.25 | 0.5 | 0.25 | 0.25 | 0.25 | 0.5 | 0.5 | 0.25 |
| *Staphylococcus aureus* V41[c] | 0.25 | 0.5 | 0.25 | 0.25 | 0.25 | 0.5 | 0.5 | 0.25 |
| *Staphylococcus aureus* X400[d] | 0.25 | 1 | 0.25 | 0.25 | 0.25 | 0.5 | 0.5 | 0.25 |
| *Staphylococcus aureus* S13E | 0.25 | 0.5 | 0.25 | 0.25 | 0.25 | 0.5 | 0.5 | 0.25 |
| *Staphylococcus epidermidis* EP11 | 0.25 | 0.5 | 0.25 | 0.25 | 0.25 | 0.5 | 0.5 | 0.25 |
| *Staphylococcus epidermidis* EP12 | 0.12 | 0.12 | 0.12 | 0.12 | 0.12 | 0.25 | 0.25 | 0.12 |
| *Streptococcus pyogenes* C203 | 2 | 1 | 1 | 1 | 0.06 | 0.5 | 0.25 | 0.12 |
| *Streptococcus pneumoniae* Park I | 2 | 1 | 1 | 1 | 0.06 | 1 | 1 | 1 |
| *Streptococcus* Group D X66 | 8 | 4 | 4 | 4 | 0.25 | 8 | 4 | 4 |
| *Streptococcus* Group 9960 | 16 | 8 | 8 | 8 | 0.5 | 16 | 8 | 8 |
| *Haemophilus influenzae* Holt[e] | 32 | 16 | 32 | 16 | 16 | 16 | 32 | 32 |
| *Haemophilus influenzae* R252[f] | 32 | 16 | 16 | 16 | 16 | 32 | 16 | 16 |

[a] MIC in mcg/ml
[b] Compound numbers from Tables I—VIII
[c] Penicillin-resistant strain
[d] Methicillin-resistant strain
[e] Ampicillin-sensitive strain
[f] Ampicillin-resistant strain
[g] Not tested
[h] Not active at 128 mcg/ml, the highest level tested

TABLE IX cont'd.
Antibiotic Activity of C—20 Modified Derivatives[a]

| Test Organism | Test Compound[b] 41 | 42 | 43 | 44 | 45 | 47 | 48 |
|---|---|---|---|---|---|---|---|
| *Staphylococcus aureus* X1.1 | 0.5 | 0.5 | 0.12 | 0.12 | 0.5 | 0.5 | 0.5 |
| *Staphylococcus aureus* V41[c] | 0.25 | 0.1 | 0.12 | 0.12 | 0.5 | 0.5 | 0.5 |
| *Staphylococcus aureus* X400[d] | 0.5 | 1 | 0.5 | 0.25 | 1 | 0.5 | 0.5 |
| *Staphylococcus aureus* S13E | 0.25 | 0.5 | 0.25 | 0.12 | 0.5 | 0.25 | 0.5 |
| *Staphylococcus epidermidis* EP11 | 0.5 | 0.5 | 0.12 | 0.12 | 0.5 | 0.5 | 0.5 |
| *Staphylococcus epidermidis* EP12 | 0.25 | 0.25 | 0.25 | NT | 0.25 | 0.25 | 0.25 |
| *Streptococcus pyogenes* C203 | 0.25 | 0.5 | 0.25 | 0.25 | 2 | 0.25 | 0.25 |
| *Streptococcus pneumoniae* Park I | 0.25 | NT[g] | 2 | 2 | 0.5 | 0.25 | 0.25 |
| *Streptococcus* Group D X66 | 0.5 | 8 | NT | 4 | 16 | 0.5 | 0.5 |
| *Streptococcus* Group 9960 | 0.5 | 8 | 4 | 8 | 8 | 1 | 1 |
| *Haemophilus influenzae* Holt[e] | 16 | 32 | 32 | 16 | 32 | 16 | 16 |
| *Haemophilus influenzae* R252[f] | 16 | 32 | 32 | 4 | 32 | 16 | 16 |

[a] MIC in mcg/ml
[b] Compound numbers from Tables I—VIII
[c] Penicillin-resistant strain
[d] Methicillin-resistant strain
[e] Ampicillin-sensitive strain
[f] Ampicillin-resistant strain
[g] Not tested
[h] Not active at 128 mcg/ml, the highest level tested

TABLE IX cont'd.
Antibiotic Activity of C—20 Modified Derivatives[a]

| Test Organism | Test Compound[b] 51a | 53 | 58 | 62 | 63 | 64 |
|---|---|---|---|---|---|---|
| *Staphylococcus aureus* X1.1 | 0.5 | 2 | 0.25 | 1 | 1 | 2 |
| *Staphylococcus aureus* V41[c] | 0.5 | 2 | 0.25 | 1 | 1 | 2 |
| *Staphylococcus aureus* X400[d] | 1 | 4 | 0.25 | 2 | 2 | 4 |
| *Staphylococcus aureus* S13E | 0.5 | 2 | 0.25 | 1 | 1 | 2 |
| *Staphylococcus epidermidis* EP11 | 0.5 | 2 | 0.25 | 1 | 1 | 2 |
| *Staphylococcus epidermidis* EP12 | 0.25 | 2 | 0.25 | 1 | 1 | 2 |
| *Streptococcus pyogenes* C203 | 0.5 | 4 | 0.06 | NT | 0.5 | 2 |
| *Streptococcus pneumoniae* Park I | NT | 32 | 0.25 | 2 | 16 | NT |
| *Streptococcus* Group D X66 | 16 | —[h] | 4 | — | — | — |
| *Streptococcus* Group 9960 | 16 | — | 8 | — | — | — |
| *Haemophilus influenzae* Holt[e] | 64 | — | 32 | — | — | — |
| *Haemophilus influenzae* R252[f] | 32 | — | 16 | — | — | — |

[a] MIC in mcg/ml
[b] Compound numbers from Tables I—VIII
[c] Penicillin-resistant strain
[d] Methicillin-resistant strain
[e] Ampicillin-sensitive strain
[f] Ampicillin-resistant strain
[g] Not tested
[h] Not active at 128 mcg/ml, the highest level tested

TABLE IX cont'd.
Antibiotic Activity of C—20 Modified Derivatives[a]

| Test Organism | Test Compound[b] 65 | 66 | 67 | 68 | 69 | 70 |
|---|---|---|---|---|---|---|
| *Staphylococcus aureus* X1.1 | 0.5 | 0.5 | 0.25 | 0.5 | 0.25 | 0.25 |
| *Staphylococcus aureus* V41[c] | 0.5 | 0.5 | 0.25 | 0.5 | 0.25 | 0.25 |
| *Staphylococcus aureus* X400[d] | 0.5 | 1 | 0.5 | 0.5 | 0.5 | 0.25 |
| *Staphylococcus aureus* S13E | 0.5 | 0.5 | 0.25 | 0.5 | 0.25 | 0.25 |
| *Staphylococcus epidermidis* EP11 | 0.25 | 0.5 | 0.25 | 0.5 | 0.25 | 0.25 |
| *Staphylococcus epidermidis* EP12 | NT | 0.25 | 0.12 | 0.12 | 0.12 | 0.12 |
| *Streptococcus pyogenes* C203 | 0.5 | 2 | 1 | 0.5 | 2 | 0.5 |
| *Streptococcus pneumoniae* Park I | 1 | 0.5 | 0.25 | 0.5 | 0.25 | 0.5 |
| *Streptococcus* Group D X66 | 8 | 8 | 4 | 4 | 8 | 2 |
| *Streptococcus* Group 9960 | 8 | 8 | 8 | 8 | 8 | 4 |
| *Haemophilus influenzae* Holt[e] | 64 | 64 | 32 | 32 | 16 | 4 |
| *Haemophilus influenzae* R252[f] | 32 | 64 | 16 | 16 | 16 | 4 |

[a] MIC in mcg/ml
[b] Compound numbers from Tables I—VIII
[c] Penicillin-resistant strain
[d] Methicillin-resistant strain
[e] Ampicillin-sensitive strain
[f] Ampicillin-resistant strain
[g] Not tested
[h] Not active at 128 mcg/ml, the highest level tested

TABLE X.
Antibiotic Activity of C—20 Modified Derivatives[a]

| Test Organism | Test Compound[b] 1 | 2 | 4 | 5 | 6 | 7 | 9 |
|---|---|---|---|---|---|---|---|
| *Staphylococcus aureus* | 6.25 | 3.12 | 3.12 | 1.56 | 3.12 | 3.12 | 6.25 |
| *Streptococcus bovis* 80 | 50 | 50 | 12.5 | — | — | 12.5 | — |
| *Pasteurella multocida* 17E[c] | —[e] | — | — | — | — | — | — |
| *Pasteurella multocida* 60A[d] | — | — | — | — | — | — | — |
| *Pasteurella hemolytica* 22C | — | — | — | — | — | — | — |
| *Mycoplasma gallisepticum* | 3.12 | 1.56 | 1.56 | 1.56 | 0.78 | 0.78 | 1.56 |
| *Mycoplasma synoviae* | 6.25 | 12.5 | NT | 3.12 | 12.5 | NT | 25 |
| *Mycoplasma hyorhinis* | — | — | 50 | — | — | 50 | — |
| *Mycoplasma hyopneumoniae* | NT | NT | NT | NT | >25 | NT | NT |

[a] MIC in mcg/ml
[b] Compound numbers from Tables I—VIII
[c] Bovine isolate
[d] Avian isolate
[e] Not active at 50 mcg/ml, the highest level tested
[f] Not tested

TABLE X cont'd.
Antibiotic Activity of C—20 Modified Derivatives[a]

| Test Organism | Test Compound[b] 11 | 12 | 13 | 14 | 15 | 16 | 17 |
|---|---|---|---|---|---|---|---|
| *Staphylococcus aureus* | 1.56 | 1.56 | 1.56 | 1.56 | 1.56 | 1.56 | 1.56 |
| *Streptococcus bovis* 80 | 3.12 | 25 | 1.56 | 25 | 6.25 | 50 | 50 |
| *Pasteurella multocida* 17E[c] | — | — | — | — | — | — | — |
| *Pasteurella multocida* 60A[d] | — | — | — | — | — | — | — |
| *Pasteurella hemolytica* 22C | — | — | — | — | — | — | — |
| *Mycoplasma gallisepticum* | 1.56 | 0.39 | 0.78 | 0.78 | 1.56 | 1.56 | 0.78 |
| *Mycoplasma synoviae* | 3.12 | 1.56 | 6.25 | 6.25 | 3.12 | 6.25 | 6.25 |
| *Mycoplasma hyorhinis* | — | 50 | 6.25 | — | 50 | 50 | — |
| *Mycoplasma hyopneumoniae* | NT | >25 | 3.12 | >25 | >25 | >25 | >25 |

[a] MIC in mcg/ml
[b] Compound numbers from Tables I—VIII
[c] Bovine isolate
[d] Avian isolate
[e] Not active at 50 mcg/ml, the highest level tested
[f] Not tested

TABLE X cont'd.
Antibiotic Activity of C—20 Modified Derivatives[a]

| Test Organism | Test Compound[b] 21 | 23 | 24 | 26 | 29 |
|---|---|---|---|---|---|
| *Staphylococcus aureus* | 0.78 | 0.19 | 0.78 | 0.39 | 0.78 |
| *Streptococcus bovis* 80 | 3.12 | 6.25 | 12.5 | 1.56 | 0.78 |
| *Pasteurella multocida* 17E[c] | 12.5 | 12.5 | 12.5 | 6.25 | 12.5 |
| *Pasteurella multocida* 60A[d] | 6.25 | 12.5 | 12.5 | 6.25 | 25 |
| *Pasteurella hemolytica* 22C | 12.5 | 25 | 12.5 | 12.5 | 25 |
| *Mycoplasma gallisepticum* | 0.39 | 0.09 | 1.56 | <0.05 | 0.39 |
| *Mycoplasma synoviae* | NT[f] | 6.25 | 0.78 | 1.56 | 1.56 |
| *Mycoplasma hyorhinis* | 50 | — | 50 | 50 | 50 |
| *Mycoplasma hyopneumoniae* | 12.5 | >25 | 12.5 | 0.78 | 12.5 |

[a] MIC in mcg/ml
[b] Compound numbers from Tables I—VIII
[c] Bovine isolate
[d] Avian isolate
[e] Not active at 50 mcg/ml, the highest level tested
[f] Not tested

TABLE X cont'd.
Antibiotic Activity of C—20 Modified Derivatives[a]

| Test Organism | Test Compound[b] 33 | 34 | 35 | 36 | 37 | 38 | 39 | 40 |
|---|---|---|---|---|---|---|---|---|
| *Staphylococcus aureus* | 0.39 | 0.39 | 0.39 | 12.5 | 0.19 | 0.78 | 6.25 | 0.39 |
| *Streptococcus bovis* 80 | 6.25 | 1.56 | 1.56 | 12.5 | 0.19 | 0.78 | 1.56 | 3.12 |
| *Pasteurella multocida* 17E[c] | 12.5 | 12.5 | 12.5 | 25 | 12.5 | 25 | 25 | 12.5 |
| *Pasteurella multocida* 60A[d] | 12.5 | 25 | 12.5 | 12.5 | 25 | 50 | 12.5 | 12.5 |
| *Pasteurella hemolytica* | 25 | 25 | 25 | 50 | 12.5 | 50 | — | 12.5 |
| *Mycoplasma gallisepticum* | 0.39 | 0.39 | 0.39 | 25 | 0.19 | 0.39 | 1.56 | <0.05 |
| *Mycoplasma synoviae* | 1.56 | 0.78 | 0.78 | — | 1.56 | 1.56 | 1.56 | 0.19 |
| *Mycoplasma hyorhinis* | 25 | 50 | — | 50 | 0.78 | 25 | 25 | 12.5 |
| *Mycoplasma hyopneumoniae* | 25 | 12.5 | 6.25 | 12.5 | 0.19 | NT | 6.25 | 12.5 |

[a] MIC in mcg/ml
[b] Compound numbers from Tables I—VIII
[c] Bovine isolate
[d] Avian isolate
[e] Not active at 50 mcg/ml, the highest level tested

TABLE X cont'd.
Antibiotic Activity of C—20 Modified Derivatives[a]

| Test Organism | Test Compound[b] 41 | 42 | 43 | 44 | 45 | 47 | 48 |
|---|---|---|---|---|---|---|---|
| *Staphylococcus aureus* | 0.19 | 1.56 | 0.78 | 0.39 | 1.56 | 0.78 | 0.78 |
| *Streptococcus bovis* 80 | 0.39 | 3.12 | 6.25 | 6.25 | 12.5 | 0.09 | 0.09 |
| *Pasteurella multocida* 17E[c] | 12.5 | 12.5 | 25 | 6.25 | 25 | 25 | 25 |
| *Pasteurella multocida* 60A[d] | 12.5 | 12.5 | 12.5 | 6.25 | 25 | 25 | 25 |
| *Pasteurella hemolytica* 22C | 25 | 12.5 | 25 | 12.5 | 50 | 25 | 25 |
| *Mycoplasma gallisepticum* | <0.05 | 0.39 | 0.78 | 0.39 | 0.39 | <0.05 | <0.05 |
| *Mycoplasma synoviae* | 0.39 | 0.195 | 0.78 | NT | 6.25 | 0.09 | 0.19 |
| *Mycoplasma hyorhinis* | 3.12 | 25 | 50 | 50 | —[e] | 6.25 | 12.5 |
| *Mycoplasma hyopneumoniae* | 3.12 | NT[f] | 12.5 | 6.25 | 6.25 | 3.12 | 1.56 |

[a] MIC in mcg/ml
[b] Compound numbers from Tables I—VIII
[c] Bovine isolate
[d] Avian isolate
[e] Not active at 50 mcg/ml, the highest level tested
[f] Not tested

TABLE X cont'd.
Antibiotic Activity of C—20 Modified Derivatives[a]

| Test Organism | Test Compound[b] 50 | 51 | 53 | 58 |
|---|---|---|---|---|
| *Staphylococcus aureus* | 1.56 | 0.78 | 0.39 | 0.39 |
| *Streptococcus bovis* 80 | 3.12 | 0.78 | 6.25 | 1.56 |
| *Pasteurella multocida* 17E[c] | 25 | 12.5 | 25 | 25 |
| *Pasteurella multocida* 60A[d] | 25 | 12.5 | 12.5 | 12.5 |
| *Pasteurella hemolytica* 22C | —[e] | — | 25 | 25 |
| *Mycoplasma gallisepticum* | ≤0.048 | ≤0.048 | <0.05 | 0.78 |
| *Mycoplasma synoviae* | 0.39 | 0.39 | 3.12 | 6.25 |
| *Mycoplasma hyorhinis* | 12.5 | 12.5 | 50 | 50 |
| *Mycoplasma hyopneumoniae* | NT[f] | NT | 0.78 | 6.25 |

[a] MIC in mcg/ml
[b] Compound numbers from Tables I—VIII
[c] Bovine isolate
[d] Avian isolate
[e] Not active at 50 mcg/ml, the highest level tested
[f] Not tested

TABLE X cont'd.
Antibiotic Activity of C—20 Modified Derivatives[a]

| Test Organism | Test Compound[b] 62 | 63 | 64 | 65 | 66 | 67 | 68 | 69 | 70 |
|---|---|---|---|---|---|---|---|---|---|
| *Staphylococcus aureus* | 3.12 | 6.25 | 3.12 | 1.56 | 0.78 | 0.78 | 0.39 | 0.78 | 0.78 |
| *Streptococcus bovis* 80 | 12.5 | 25 | 12.5 | 6.25 | 6.25 | 12.5 | 6.25 | 6.25 | 6.25 |
| *Pasteurella multocida* 17E[c] | —[e] | — | — | 25 | 25 | 25 | 25 | 25 | 12.5 |
| *Pasteurella multocida* 60A[d] | — | — | — | 12.5 | 25 | 25 | 25 | 12.5 | 12.5 |
| *Pasteurella hemolytica* 22C | — | — | — | 25 | 25 | 25 | 25 | 25 | 50 |
| *Mycoplasma gallisepticum* | 1.56 | 1.56 | 1.56 | <0.05 | 0.09 | 0.39 | 0.39 | 0.39 | 0.39 |
| *Mycoplasma synoviae* | NT[f] | NT | NT | NT | NT | 3.12 | 3.12 | NT | 0.78 |
| *Mycoplasma hyorhinis* | 50 | 50 | 50 | — | 50 | 50 | — | 50 | 12.5 |
| *Mycoplasma hyopneumoniae* | NT | NT | NT | 12.5 | 25 | 12.5 | 12.5 | 12.5 | 6.25 |

[a] MIC in mcg/ml
[b] Compound numbers from Tables I—VIII
[c] Bovine isolate
[d] Avian isolate
[e] Not active at 50 mcg/ml, the highest level tested
[f] Not tested

The C—20 modified derivatives of this invention have shown *in vivo* antimicrobial activity against experimentally-induced infections in laboratory animals. When two doses of test compound were administered to mice experimentally infected with *S. pyogenes* C203, the activity observed was measured as an $ED_{50}$ value [effective dose in mg/kg to protect 50% of the test animals: see Warren Wick, et al., *J. Bacteriol. 81*, 233—235 (1961)]. $ED_{50}$ values observed for illustrative compounds are given in Table XI.

TABLE XI
$ED_{50}$ Values of C—20-Modified Derivatives[a]

| | *Streptococcus pyogenes* C203 | |
|---|---|---|
| Test Compound[b] | Subcutaneous | Oral |
| 1 | >6.3 | 17 |
| 2 | >6.3 | 18 |
| 4 | >6.3 | 35 |
| 5 | >6.3 | 56 |
| 7 | >6.3 | 11 |
| 21 | 0.7 | 6 |
| 23 | 10.0 | 17 |
| 24 | 2.4 | 50 |
| 26 | 2.4 | >25 |
| 29 | 3.4 | 36 |
| 33 | 1.5 | 9 |
| 34 | 1.1 | 5 |
| 35 | 1.6 | 11 |
| 36 | 2.2 | 19 |
| 37 | 7.2 | 18 |
| 38 | >10 | 35 |
| 39 | 6.7 | 19 |
| 40 | >10 | 31 |
| 41 | >10 | 25 |
| 42 | >25 | >100 |
| 43 | 1.6 | 16 |
| 44 | 3.8 | 13 |
| 45 | 2.1 | >50 |
| 47 | >10 | 29 |
| 48 | >10 | 35 |
| 58 | 3.2 | 50 |

TABLE XI cont'd.
$ED_{50}$ Values of C—20-Modified Derivatives[a]

| | *Streptococcus pyogenes* C203 | |
|---|---|---|
| Test Compound[b] | Subcutaneous | Oral |
| 62 | >6.3 | 66 |
| 63 | >6.3 | 22 |
| 65 | 1.7 | 20 |
| 66 | >6.3 | 31 |
| 67 | 1.0 | 9 |
| 68 | 2.9 | 16 |
| 69 | 3.3 | 27 |
| 70 | 3.1 | 7 |

[a] mg/kg × 2; doses given 1 and 4 hours post-infection
[b] Compound numbers from Tables I—VIII
[c] Not tested

Many of the C—20 modified derivatives of this invention have also shown *in vivo* antibacterial activity against infections induced by gram-negative bacteria. Tables XII and XIII summarize the results of tests in which illustrative compounds were evaluated against *Pasteurella* infection in one-day-old chicks. The compounds were administered parenterally or orally after challenge of the chicks with *Pasteurella multocida* (0.1 ml of a 10—4 dilution of a twenty-hour tryptose broth culture of an avian *P. multocida* given subcutaneously). In these tests, unless indicated otherwise, all non-medicated infected chicks died within 24 hours of *Pasteurella* challenge. In the tests summarized in Table XII the compounds were administered by subcutaneous injection at a dosage of 30 mg/kg, 1 and 4 hours post-challenge of the chicks with *P. multocida*. In the tests summarized in Table XIII the compounds were administered by gavage at 1 and 5 hours post-challenge of the chicks with *P. multocida*.

TABLE XII
Activity of C—20-Modified Derivatives
Administered Subcutaneously to
*Pasteurella multocida*-Infected Chicks[a]

| Test Compound[b] | Number of Deaths/Number Treated |
|---|---|
| 21 | 9/10 |
| 21 | 7/10[c] |
| 24 | 2/10 |
| 24 | 0/10 |
| 26 | 8/10 |
| 29 | 5/10 |
| 33 | 10/10 |
| 43 | 10/10 |
| 44 | 9/10 |
| 45 | 9/10 |

[a] Administered subcutaneously; 30 mg/kg × 2
[b] Compound numbers from Tables I—VIII
[c] 8/10 infected non-medicated chicks died in this test
[d] 8/10 and 9/10 of two groups of infected non-medicated chicks died in this test

TABLE XIII
Activity of C—20-Modified Derivatives Administered Orally
to *Pasteurella multocida*-Infected Chicks[a]

| Test Compound[b] | Dose (g/gal)[c] | Number of Deaths/ Number tested |
|---|---|---|
| 21 | 2.0 | 0/9 |
| 21 | 1.0 | 3/9 |
| 21 | 2.0 | 0/10 |
| 21 | 2.0 | 2/10[d] |
| 22 | 0.5 | 6/10 |
| 22 | 1.0 | 0/10 |
| 22 | 2.0 | 1/10 |
| 23 | 0.5 | 8/10 |
| 23 | 1.0 | 10/10 |
| 24 | 2.0 | 1/9 |
| 24 | 1.0 | 2/9 |
| 29 | 0.5 | 0/10 |
| 29 | 1.0 | 3/10 |

TABLE XIII cont'd.

| Test Compound[b] | Dose (g/gal)[c] | Number of Deaths/ Number tested |
|---|---|---|
| 33 | 2.0 | 3/9 |
| 33 | 1.0 | 9/9 |
| 33 | 2.0 | 7/10[d] |
| 34 | 0.5 | 7/10 |
| 34 | 1.0 | 9/10 |
| 35 | 1.5 | 1/10 |
| 35 | 2.0 | 4/10 |
| 37 | 1.5 | 10/10 |
| 40 | 1.5 | 10/10 |

[a] Administered by gavage

[b] Compound number from Tables I—VIII

[c] Dose administered in 0.1-ml of solution equivalent to the presumed amount of compound which would be consumed by chicks drinking medicated water at the concentration indicated over a 24-hour period

[d] 13/20 Infected non-medicated chicks died in this test

[e] 11/20 Infected non-medicated chicks died in this test

The compounds of this invention have also exhibited *in vivo* activity against experimental infections caused by *Mycoplasma gallisepticum*. In these tests infections were induced in chicks by injecting 0.2 ml of a broth culture of *M. gallisepticum* into the abdominal air sac of two- to three-day-old chicks. The compounds were administered by gavage five times at a dose equivalent to 0.5 g/gal (on the day of challenge once prior to and once following the challenge, two times on the next day and once on the third day). Twenty-one days post-infection, the chicks were weighed, a blood sample was taken, and the chicks were sacrificed. The presence or absence of air-sac lesions was recorded. The results of these tests are summarized in Table XIV.

TABLE XIV
Antimycoplasmal Activity of C—20-Modified Derivatives in Chicks

| Test Compound[a] | Mortality | Number with Air-Sac Lesions/ Number Treated | Number with Antibodies[b]/ Number Tested |
|---|---|---|---|
| 24 | 0/10 | 6/10 | 10/10 |
| 26 | 1/10 | 5/10 | 7/9 |
| 33 | 0/10 | 2/10 | 10/10 |
| 44 | 0/10 | 7/10 | 9/10 |
| Infected Control | 2/10 | 10/10 | 8/8 |
| Normal Control | 0/0 | 0/0 | 0/0 |

[a] Compound numbers from Tables I—VIII
[b] Antibodies to *M. gallisepticum*

This invention is useful in methods of controlling infections caused by bacterial and mycoplasmal species. In carrying out these methods, an effective amount of a compound of formula I is administered parenterally or orally to an infected or susceptible warm-blooded animal. The compounds can also be administered by insufflation, i.e. by blowing the compound, in the form of a medicated dust, into an enclosed space or room where the animals or poultry are held. The animals or poultry breathe the medicated dust present in the air; the medicated dust is also taken into the body through the eyes (a process called intraocular injection).

The dose which is effective to control the infection will vary with the severity of the infection and the age, weight, and condition of the animal. The total dose required for protection parenterally will generally, however, be in the range of from about 1 to about 100 mg/kg and preferably will be in the range of from about 1 to about 50 mg/kg. The dose required for oral administration will generally be in the range of from 1 to about 300 mg/kg and preferably will be in the range of from about 1 to about 100 mg/kg. Suitable dosage regimens can be constructed.

Often the most practical way to administer the compounds is by formulation into the feed supply or drinking water. A variety of feeds, including the common dry feeds, liquid feeds, and pelleted feeds, may be used.

In another aspect, this invention relates to compositions useful for the control of infections caused by bacteria and *Mycoplasma* species. These compositions comprise a compound of formula *I* together with a suitable vehicle. Compositions may be formulated for parenteral or oral administration by methods recognized in the pharmaceutical art.

The methods of formulating drugs into animal feeds are well-known. A preferred method is to make a concentrated-drug premix which in turn is used to prepare medicated feeds. Typical premixes may contain from about 1 to about 200 grams of drug per pound of premix. Premixes may be either liquid or solid preparations.

The final formulation of feeds for animals or poultry will depend upon the amount of drug to be administered. The common methods of formulating, mixing, and pelleting feeds may be used to prepare feeds containing a compound of formula *I*.

Effective injectable compositions containing these compounds may be either suspension or solution form. In the preparation of suitable formulations it will be recognized that, in general, the water solubility of the acid addition salts is greater than that of the free bases. Similarly, the bases are more soluble in dilute acids or in acidic solutions than in neutral or basic solutions.

In the solution form the compound is dissolved in a physiologically acceptable vehicle. Such vehicles comprise a suitable solvent, preservatives such as benzyl alcohol, if needed, and buffers. Useful solvents include, for example, water and aqueous alcohols, glycols, and carbonate esters such as diethyl carbonate. Such aqueous solutions contain, in general, no more than 50% of the organic solvent by volume.

Injectable suspension compositions require a liquid suspending medium, with or without adjuvants, as a vehicle. The suspending medium can be, for example, aqueous polyvinylpyrrolidone, inert oils such as vegetable oils or highly refined mineral oils, or aqueous carboxymethylcellulose.

Suitable physiologically acceptable adjuvants are necessary to keep the compound suspended in suspension compositions. The adjuvants may be chosen from among thickeners such as carboxymethylcellulose, polyvinylpyrrolidone, gelatin, and the alginates. Many surfactants are also useful as suspending agents. Lecithin, alkylphenol polyethylene oxide adducts, naphthalenesulfonates, alkylbenzenesulfonates,

and the polyoxyethylene sorbitan esters are useful suspending agents.

Many substances which affect the hydrophilicity, density, and surface tension of the liquid suspending medium can assist in making injectable suspensions in individual cases. For example, silicone antifoams, sorbiol, and sugars can be useful suspending agents.

In order to illustrate more fully the operation of this invention, the following examples are provided. In these examples the abbreviations "20-DH" and "20-DH-DO" are used for the terms "20-dihydro" and "20-dihydro-20-deoxy", respectively.

Preparation 1

20-Dihydrotylosin (Relomycin)

A solution of tylosin base (30.0 g, 32.8 mmole) in 2-propanol (300 ml) and water (200 ml) was treated with sodium borohydride (315 mg, 8.2 mmole), portionwise, over five minutes. Thirty minutes after the addition was completed, the pH of the reaction solution was adjusted to 7.0 by the addition of a 1N sulfuric acid solution. The neutralized solution was evaporated under vacuum to remove the 2-propanol; the aqueous solution remaining was treated with a saturated sodium bicarbonate solution (500 ml). The mixture was extracted with dichloromethane (3 × 300 ml), and the combined extract was washed with a saturated sodium chloride solution (1 × 200 ml) and dried over sodium sulfate. Filtration followed by evaporation gave a glass which was broken up in *n*-hexane, collected on a filter and air dried to yield 28.5 g (95%) of 20-dihydrotylosin.

Preparation 2

20-Dihydrodesmycosin

Desmycosin (10 g, 13 mmoles), dissolved in isopropanol:water (1:1, 175 ml), was stirred at room temperature while $NaBH_4$ (125 mg, 3.3 mmoles) was added. After 1/2 hour the pH of the reaction mixture was adjusted to 7.0 with 1*N* $H_2SO_4$. The alcohol was removed under reduced pressure. Saturated $NaHCO_3$ solution was added to the aqueous solution, and the product was extracted into $CH_2Cl_2$. The organic layer was dried ($Na_2SO_4$), and solvent was removed under reduced pressure to give 9.65 g of 20-dihydrodesmycosin (12.5 mmoles, 96% yield) as a white foam.

Preparation 3

20-DH-DO-20-iododesmycosin (Method 1)

20-Dihydrodesmycosin (2.0 g, 2.6 mmoles) and tetra n-butylammonium iodide (1.5 g, 3.9 mmoles) were dissolved in $CH_2Cl_2$ (30 ml) with s-collidine (0.6 ml, 4.5 mmoles) added. This solution was cooled to −78°C under a nitrogen atmosphere and treated with trifluoromethanesulfonic anhydride (0.6 ml, 3.9 mmoles) dropwise by syringe. The reaction was stirred for 5 minutes at −78°C and then allowed to come to room temperature (about 30 minutes). Saturated $NaHCO_3$ solution was added, and the product was extracted with $CH_2Cl_2$. The organic layer was dried ($Na_2SO_4$) and evaporated to give a red oil which was purified by silica-gel flash chromatography, eluting initially with $CH_2Cl_2$ (400 ml) and then stepwise with $CH_2Cl_2$:$CH_3OH$ solutions as follows: 98:2 (250 ml); 96:4 (500 ml); 95:5 (250 ml); 94:6 (750 ml) and 92:8 (250 ml). Fractions containing the desired product were identified by TLC, combined and evaporated to dryness to give 20-DH-CO-20-iododesmycosin (595 mg, 0.67 mmoles, 26% yield) as a white foam.

Preparation 4

20-DH-DO-20-iododesmycosin (Method 2)

20-Dihydrodesmycosin (5.0 g, 6.5 mmoles) and triphenylphosphine (2.54 g, 9.70 mmoles) were dissolved in dimethylformamide (DMF) (10 ml). This mixture was stirred at room temperature under $N_2$ while iodine (2.46 g, 9.70 mmoles) in DMF (5 ml) was added dropwise. The reaction mixture was stirred for two hours and then poured into cold saturated $NaHCO_3$ solution. The product was extracted with $CHCl_3$ (two portions) and the combined $CHCl_3$ extracts were shaken with 0.1*M* sodium thiosulfate to remove unreacted iodine. The organic layer was dried ($Na_2SO_4$) and evaporated under reduced pressure to give a light yellow oil which was purified by silica-gel flash chromatography. The column was eluted initially with $CH_2Cl_2$ (500 ml) and then with 250 ml portions of $CH_2Cl_2$:$CH_3OH$ mixtures as follows: 98:2; 96:4; 95:5; 94:6; 92:8; 88:12; and 86:14. Fractions containing the desired product were identified as in Preparation 2 and combined to give 1.78 g (2.0 mmoles, 31% yield) of 20-DH-DO-20-iododesmycosin as a white foam.

Preparation 5

20-DH-20-O-(*p*-Toluenesulfonyl)tylosin

A solution of 20-dihydrotylosin (10.0 g, 10.9 mmole) and 4-(N,N-dimethylamino)pyridine (24 mg, 0.2 mmole) in dichloromethane (100 ml) and pyridine (10 ml) was treated with *p*-toluenesulfonyl chloride (2.08 g, 10.9 mmole). The resulting solution was stirred at room temperature with the exclusion of moisture. Additional *p*-toluenesulfonyl chloride was added after three hours (1.0 g, 5.2 mmole) and after twenty-two hours (240 mg, 1.3 mmole). After twenty-seven hours, methanol (0.8 ml) was added, and the solution was evaporated to give a glass. The glass was dissolved in dichloromethane, washed with saturated sodium bicarbonate solution and dried over sodium sulfate. The solution was filtered and then evaporated to give a glass that was purified by silica-gel flash chromatography. Elution with a gradient of 1 L of

dichloromethane to 1 L of methanol/dichloromethane (3.5:96.5) and then with 2 L of methanol/dichloromethane (3.5:96.5) gave 5.37 g (46%) of pure 20-DH-DO-20-O-(*p*-toluenesulfonyl)tylosin and 2.3 g (20%) of slightly impure 20-DH-20-O-(*p*-toluenesulfonyl)tylosin.

Example 1

20-DH-DO-20-N-Phthalimidotylosin

A solution of 20-dihydrotylosin (3.,0 g, 3.27 mmole), triphenylphosphine (1.714 g, 6.54 mmole) and phthalimide (962 mg, 6.54 mmole) in tetrahydrofuran (50 ml) under argon was treated dropwise, over one minute, with diethyl azodicarboxylate (1.03 ml, 6.54 mmole). Thirty minutes after the addition was completed, methanol (0.3 ml) was added; after ten minutes, the reaction was evaporated under vacuum to give a glass. This glass was purified by silica-gel flash chromatography, eluting with dichloromethane (300 ml) and then gradients of 1 L of dichloromethane to 1 L of methanol/dichloromethane (3:97) and 1 L of methanol/dichloromethane (3:97) to 1 L of methanol/dichloromethane (6:94), to give 2.35 g (70%) of 20-DH-DO-20-N-phthalimidotylosin.

Example 2

20-DH-DO-20-N-Phthalimidodesmycosin

A solution of 20-DH-DO-20-N-phthalimidotylosin (1.04 g, 1.0 mmole) in 1N sulfuric acid (50 ml) was stirred at room temperature for one hour. The reaction solution was carefully added to a saturated sodium bicarbonate solution (100 ml), and the resulting mixture was extracted with dichloromethane (3 × 30 ml). The combined extract was dried over sodium sulfate. Filtration followed by evaporation yielded 890 mg (99%) of 20-DH-DO-20-N-phthalimidodesmycosin.

Example 3

20-DH-DO-20-[(1-N-Methyltetrazol-5-yl)thio]tylosin

A solution of 20-dihydrotylosin (3.0 g, 3.27 mmole) and triphenylphosphine (1.714 g, 6.54 mmole) in tetrahydrofuran (50 ml) under argon was treated dropwise, over one minute, with diethyl azodicarboxylate (1.05 ml, 6.54 mmole). One minute after the addition was completed, 5-mercapto-1-N-methyltetrazole (760 mg, 6.54 mmole) was added in one portion. Thirty minutes later, methanol (1 ml) was added, and the reaction solution was evaporated. The residue obtained was dissolved in ethyl acetate and extracted three times with 0.1 M acetic acid solution. The acidic extracts were combined and saturated by the careful addition of solid sodium bicarbonate. The resulting mixture was extracted three times with dichloromethane. The extracts were combined and dried over sodium sulfate. The solution was filtered and then evaporated to give a glass. The glass was purified by silica-gel flash chromatography, eluting first with dichloromethane (300 ml) and then with a gradient of 1 L of dichloromethane to 1 L of methanol/dichloromethane (3:97) followed by one of 1 L of methanol/dichloromethane (3:97) to 1 L of methanol/dichloromethane (1:9), to give 2.24 g (67%) of 20-DH-DO-20-[(1-N-methyltetrazol-5-yl)thio]tylosin.

Example 4

20-DH-DO-20-[(1-N-Methyltetrazol-5-yl)thio]desmycosin

20-DH-DO-20-[(1-N-methyltetrazol-5-yl)thio]tylosin (1.2 g, 1.18 mmole) was treated, using a procedure like that of Example 2, to give 920 mg (89%) of 20-DH-DO-20-[(1-N-methyltetrazol-5-yl)thio]desmycosin.

Example 5

20-DH-DO-20-Chlorotylosin

A solution of 20-dihydrotylosin (3.0 g, 3.27 mmole), triphenylphosphine (2.57 g, 9.8 mmole) and carbon tetrachloride (0.48 ml, 4.9 mmole) in dichloromethane (60 ml) and pyridine (6 ml) was stirred at room temperature, under argon, for sixty-four hours. The reaction was treated with methanol (1 ml) and stirred for thirty minutes before being evaporated to give a glass. The glass was dissolved in dichloromethane, diluted with an equal volume of cyclohexane and evaporated. This process was repeated several times. The resulting pyridine-free glass was purified by silica-gel flash chromatography, eluting with dichloromethane (300 ml) and then a gradient of 1 L of dichloromethane to 1 L of methanol/dichloromethane (7:93), to give 2.06 g (67%) of 20-DH-DO-20-chlorotylosin.

Example 6

20-DH-DO-20-Chlorodesmycosin

20-Chlorotylosin (935 mg, 1.0 mmole) was treated, using a procedure like that of Example 2, to give 790 mg (100%) of 20-DH-DO-20-chlorodesmycosin.

Example 7

20-DH-20-O-Phenyltylosin

Using a procedure like that described in Example 1, 20-dihydrotylosin (3.0 g, 3.27 mmole), triphenylphosphine (1.714 g, 6.54 mmole), phenol (615 mg, 6.54 mmole) and diethyl azodicarboxylate (1.05 ml, 6.54 mmole) were reacted to give a crude glass. The glass was purified by silica-gel chromatography on a Waters Prep 500, eluting first with 1 L of dichloromethane, then with a gradient of 2 L of dichloromethane to

2 L of methanol/dichloromethane (5:95) and finally with 2 L of methanol/dichloromethane (5:95), to give 2.07 g (64%) of 20-DH-20-O-phenyltylosin.

Example 8

20-DH-20-O-Phenyldesmycosin

20-DH-20-O-Phenyltylosin (1.2 g, 1.2 mmole) was treated, using a procedure like that of Example 2, to give 1.02 g (100%) of 20-DH-20-O-phenyldesmycosin.

Example 9

20-DH-20-O-(Phenoxyacetyl)tylosin

A solution of 20-dihydrotylosin (5.0 g, 5.45 mmole) in dichloromethane (50 ml) and pyridine (3 ml) at 0°C. was treated dropwise, over fifteen minutes, with a solution of phenoxyacetyl chloride (0.75 ml, 5.45 mmole) in dichloromethane (5 ml). Additional phenoxyacetyl chloride was added after forty-five minutes (0.25 ml, 1.82 mmole) and after sixty-five minutes (0.25 ml, 1.82 mmole). After two hours, methanol (1 ml) was added. The reaction was stirred for fifteen minutes, and then washed with a saturated sodium bicarbonate solution and dried over sodium sulfate. The solution was filtered and evaporated to give a glass. The glass was purified by silica-gel flash chromatography, eluting with dichloromethane (100 ml), a gradient of 1 L of dichloromethane to 1 L of methanol/dichloromethane (3.5:96.5) and then 1 L of methanol/dichloromethane (3.5:96.5) to give 3.4 g (59%) of 20-DH-20-O-(phenoxyacetyl)tylosin.

Example 10

20-DH-20-O-(Phenoxyacetyl)desmycosin

20-DH-20-O-(Phenoxyacetyl)tylosin (1.4 g, 1.3 mmole) was treated, using a procedure like that in Example 2, to give 1.0 g (83%) of 20-DH-20-O-(phenoxyacetyl)desmycosin.

Example 11

20-DH-DO-20-Azidotylosin

A solution of 20-dihydrotylosin (9.17 g, 10.0 mmole) and triphenylphosphine (5.24 g, 20.0 mmole) in tetrahydrofuran (200 ml) under argon at 0°C. was treated dropwise, over one minute, with diethyl azodicarboxylate (3.3 ml, 20 mmole). Five minutes after the addition was complete, the reaction was treated with diphenylphosphoryl azide (4.31 ml, 20 mmole), dropwise, over five minutes. Ten minutes after this addition was complete, the cooling bath was removed; thirty-five minutes later, methanol (2 ml) was added. Thirty minutes later the reaction solution was evaporated. The residue obtained was dissolved in dichloromethane, washed with saturated sodium bicarbonate solution and dried over sodium sulfate. The solution was filtered and evaporated to give a glass. The glass was purified by silica-gel chromatography on a Waters Prep 500, eluting first with 2 L of dichloromethane and then with gradients of 4 L of dichloromethane to 4 L of methanol/dichloromethane (5:95) to 2 L of methanol/dichloromethane (5:95) to 2 L of methanol/dichloromethane (7.5:92.5), to give 5.23 g (56%) of 20-DH-DO-20-azidotylosin.

Example 12

20-DH-DO-20-Azidodesmycosin

20-DH-DO-20-Azidotylosin (3.25 g, 3.45 mmole) was treated, using a procedure like that of Example 2, to give 2.75 g (100%) of 20-DH-DO-20-azidodesmycosin.

Example 13

20-DH-DO-20-N-Phthalimido-2'-O-propionyltylosin

A solution of 20-DH-DO-20-N-phthalimidotylosin (600 mg, 0.57 mmole) in acetone (10 ml) was treated with propionic anhydride (0.15 ml, 1.15 mmole) and stirred at room temperature with the exclusion of moisture. After fifteen hours the reaction was treated with additional propionic anhydride (0.07 ml, 0.54 mmole). Twenty-four hours later, the reaction was poured into saturated sodium bicarbonate solution (50 ml). The resulting mixture was extracted three times with dichloromethane. The extracts were combined, dried over sodium sulfate, filtered, and evaporated to give a quantitative yield of 20-DH-DO-20-N-phthalimido-2'-O-propionyltylosin.

Example 14

20-DH-DO-20-N-Phthalimido-2'-O-propionyldesmycosin

A solution of 20-DH-DO-20-N-phthalimido-2'-O-propionyltylosin (490 mg, 0.44 mmole) in 1N sulfuric acid (10 ml) and dioxane (1 ml) was stirred at room temperature for one hour. The reaction mixture was carefully saturated with solid sodium bicarbonate and extracted three times with dichloromethane. The extracts were combined, dried over sodium sulfate, filtered, and evaporated to give a quantitative yield of 20-DH-DO-20-N-phthalimido-2'-O-propionyldesmycosin.

Example 15

20-DH-DO-20-N-Phthalimido-2',4'-di-O-propionyldesmycosin

A solution of 20-DH-DO-20-N-phthalimido-2'-O-propionyldesmycosin (220 mg, 0.23 mmole) in acetone

(8 ml) was treated with propionic anhydride (0.06 ml, 0.46 mmole) and stirred at room temperature with the exclusion of moisture for one week. The reaction was poured into saturated sodium bicarbonate solution (40 ml), and the resulting mixture was extracted three times with dichloromethane. The extracts were combined, dried over sodium sulfate, filtered and evaporated to give 220 mg (94%) of 20-DH-DO-20-N-phthalimido-2',4'-di-O-propionyldesmycosin.

Example 16

20-DH-20-O-Phenyl-2'-O-acetyltylosin

A solution of 20-DH-20-O-phenyltylosin (5.0 g, 5.04 mmole) in acetone (90 ml) was treated with acetic anhydride (2.0 ml, 21.2 mmole) and stirred at room temperature with the exclusion of moisture for sixteen hours. The reaction mixture was evaporated to one-half volume and poured into a saturated sodium bicarbonate solution (200 ml). The resulting mixture was extracted three times with dichloromethane. The extracts were combined, dried over sodium sulfate, filtered and evaporated to give a quantitative yield of 20-DH-20-O-phenyl-2'-O-acetyltylosin.

Example 17

20-DH-20-O-Phenyl-2'-O-acetyldesmycosin

20-DH-20-O-Phenyl-2'-O-acetyltylosin (4.5 g, 4.35 mmole) was treated, using a procedure like that in Example 2, to give a crude glass. The glass was purified by silica-gel flash chromatography, eluting with dichloromethane (300 ml) and then a gradient of 1 L of dichloromethane to 1 L of methanol/dichloromethane (5:95), to give 2.9 g (75%) of 20-DH-20-O-phenyl-2'-O-acetyldesmycosin.

Example 18

20-DH-20-O-Phenyl-2',4'-di-O-acetyldesmycosin

A solution of 20-DH-20-O-phenyl-2'-O-acetyldesmycosin (610 mg, 0.69 mmole) in acetone (20 ml) was treated with acetic anhydride (0.40 ml, 4.03 mmole) and stirred at room temperature with the exclusion of moisture for forty hours. The reaction mixture was treated, using a procedure like that described in Example 15, to give 625 mg (98%) of 20-DH-20-O-phenyl-2',4'-di-O-acetyldesmycosin.

Example 19

20-DH-20-O-Chloro-2'-O-propionyltylosin

A solution of 20-DH-DO-20-chlorotylosin (770 mg, 0.82 mmole) in acetone (20 ml) was treated with propionic anhydride (0.3 ml, 2.5 mmole) and stirred at room temperature with the exclusion of moisture for three days. A work-up like that in Example 15 gave a glass. The glass was purified by silica-gel flash chromatography, eluting with a gradient of 500 ml of dichloromethane to 500 ml of methanol/dichloromethane (4:96), to give 380 mg (47%) of 20-DH-DO-20-chloro-2'-O-propionyltylosin.

Example 20

20-DH-DO-20-Chloro-2'-O-propionyldesmycosin

A solution of 20-DH-DO-20-chloro-2'-O-propionyltylosin (250 mg, 0.25 mmole) in 1N sulfuric acid (25 ml) was stirred for one hour at room temperature. A work-up like that in Example 17 gave 209 mg (98%) of 20-DH-DO-20-chloro-2'-O-propionyldesmycosin.

Example 21

20-DH-DO-20-[N-(Ethoxycarbonyl)-N-(ethoxycarbonylamino)]aminotylosin

A solution of 20-dihydrotylosin (3.0 g, 3.27 mmole) and triphenylphosphine (1.286 g, 4.91 mmole) in dichloromethane (50 ml) under argon was treated with diethylazodicarboxylate (0.81 ml, 4.91 mmole) all at once. After the reaction mixture was stirred for thirty minutes, additional triphenylphosphine (429 mg, 1.64 mmole) and diethylazodicarboxylate (0.27 ml, 1.64 mmole) were added. One hour later, methanol (1 ml) was added, and the reaction mixture was evaporated to give a glass. The glass was purified by silica-gel flash chromatography, eluting with a gradient of 1 L of dichloromethane to 1 L of methanol/dichloromethane (4:96) and then with 1 L of methanol/dichloromethane (4:96), to give 2.0 g (57% yield) of 20-DH-DO-20-[N-(ethoxycarbonyl)-N-(ethoxycarbonylamino)]aminotylosin.

Example 22

20-DH-DO-20-[N-(Ethoxycarbonyl)-N-(ethoxycarbonylamino)]aminodesmycosin

This compound was prepared from 20-DH-DO-20-[N-ethoxycarbonyl)-N-(ethoxycarbonylamino)]-aminotylosin as described in Example 2.

Example 23

20-DH-20-O-(p-Formylphenyl)desmycosin

A solution of 20-DH-desmycosin (15.4 g, 19.9 mmole), triphenylphosphine (10.44 g, 39.8 mmole) and p-hydroxybenzaldehyde (4.86 g, 39.8 mmole) in tetrahydrofuran (300 ml) under argon was treated with diethylazodicarboxylate (6.3 ml, 39.8 mmole) all at once. After 1.3 hours, methanol (15 ml) was added, and the reaction mixture was evaporated. The resulting glass was purified by silica-gel chromatography on a

Waters Prep 500, eluting first with 2 L of dichloromethane, then with a gradient of 4 L of dichloromethane to 4 L of methanol/dichloromethane (1:7) and finally with 2 L of methanol/dichloromethane (1:7) to give 10.19 g (58% yield) of 20-DH-20-O-(*p*-formylphenyl)desmycosin.

Example 24 (Intermediate)

20-DH-20-O-Phenyl-2′,4′-di-O-acetyl-4″,3-di-O-(tetrahydropyran-2-yl)desmycosin

A solution of 20-DH-20-O-phenyl-2′,4′-di-O-acetyl-desmycosin (3.53 mmole) of dichloromethane (50 ml) was treated with distilled dihydropyran (3.4 ml, 37.2 mmole) and pyridinium p-toluenesulfonate (1.33 g, 5.3 mmole) and refluxed under argon overnight. The reaction mixture was washed with sodium chloride solution and then with sodium bicarbonate solution, dried over sodium sulfate, filtered, and evaporated to give a quantitative yield of 20-DH-20-O-phenyl-2′,4′-di-O-acetyl-4″,3-di-O-(tetrahydropyran-2-yl)-desmycosin as a mixture of diastereomers.

Example 25 (Intermediate)

20-DH-20-O-Phenyl-4″,3-di-O-(tetrahydropyran-2-yl)desmycosin

A solution of 20-DH-20-O-phenyl-2′,4′-di-O-acetyl-4″,3-di-O-(tetrahydropyran-2-yl)desmycosin (3.53 mmole) in methanol (50 ml) was heated at 50°C under argon overnight. The reaction mixture was evaporated to give a quantative yield of 20-DH-20-O-phenyl-4″,3-di-O-(tetrahydropyran-2-yl)desmycosin as a mixture of diastereomers.

Example 26

20-DH-20-O-Phenyl-4″,3-di-O-THP-4′-iodo-desmycosin

A solution of 20-DH-20-O-phenyl-4″,3-di-O-(tetrahydropyran-2-yl)desmycosin (660 mg, 0.65 mmole) in pyridine was cooled to −35°C under argon and treated with benzylsulfonyl chloride (191 mg, 1.0 mmole). After one hour, water (0.024 ml) was added; five minutes later, the cold reaction mixture was evaporated to a small volume, taken up in toluene and evaporated to 20% of the original volume. This mixture was treated with a saturated sodium bicarbonate solution. The organic layer was separated, dried over sodium sulfate, filtered and evaporated, finally from toluene (2 times) to give an oil (avoid extended evaporation). This oil was dissolved in methyl ethyl ketone (20 ml), treated with sodium iodide (250 mg, 1.67 mmole) and refluxed under argon twenty-five minutes. The cooled mixture was evaporated, dissolved in toluene, and filtered. The filtrate was washed with 1) a mixture of saturated sodium bicarbonate solution (20 ml) and 0.1 M sodium thiosulfate solution (10 ml), 2) water, and 3) saturated sodium bicarbonate solution and then was dried over sodium sulfate, filtered and evaporated. The crude product thus obtained was purified by silica-gel flash chromatography, eluting with a gradient of 0.5 L toluene to 0.5 L ethyl acetate to give 470 mg (64% yield) of 20-DH-20-O-phenyl-4″,3-di-O-THP-4′-iodo-desmycosin as a mixture of diastereomers.

Example 27

20-DH-20-O-Phenyl-2′-O-acetyl-4′-deoxydesmycosin

A solution of 20-DH-20-O-phenyl-4″,3-di-O-(tetrahydropyran-2-yl)-4′-iodo-desmycosin (460 mg, 0.41 mmole) in benzene (15 ml) was treated with tri-n-butyltin hydride (0.11 ml, 0.41 mmole) and a catalytic amount of 2,2′-azobis(2-methylpropionitrile) (AIBN) and refluxed under argon for one hour. The reaction mixture was evaporated to an oil and treated with 1N sulfuric acid solution (20 ml), n-hexane (10 ml) and tetrahydrofuran (30 ml). The resulting mixture was stirred for two hours and then was evaporated to aqueous and saturated by the addition of solid sodium bicarbonate. This solution was extracted with dichloromethane, and the combined extract was dried over sodium sulfate, filtered and evaporated to give an oil which was dissolved in acetonitrile and extracted with n-hexane (2 times). The acetonitrile solution was evaporated to a glass. To facilitate chromatographic purification, this glass was dissolved in acetone and treated with acetic anhydride (0.2 ml, 2.12 mmole) for 1.5 hours. The reaction mixture was worked up as in Example 15 to give a glass (370 mg) which was purified by silica-gel flash chromatography. Elution with 0.6 L of dichloromethane to 0.6 L of methanol/dichloromethane (1:9) gave 155 mg (43% yield) of 20-DH-20-O-phenyl-2′-O-acetyl-4′-deoxydesmycosin.

Example 28

20-DH-20-O-Phenyl-4′-deoxydesmycosin

A solution of 20-DH-20-O-phenyl-2′-O-acetyl-4′-deoxydesmycosin (130 mg, 0.15 mmole) in methanol was heated at 55°C for 3.25 hours. The reaction mixture was evaporated to give a quantitative yield of 20-DH-20-O-phenyl-4′-deoxydesmycosin.

Example 29

20-DH-20-O-[*p*-(Hexahydroazepin-1-ylmethyl)phenyl]desmycosin

A solution of 20-DH-20-O-(p-formylphenyl)desmycosin (2.43 g, 2.78 mmole) and hexamethyleneimine (0.94 ml, 8.33 mmole) in methanol (25 ml) under argon was treated with 3A molecular sieves (3 g). After thirty minutes sodium cyanoborohydride (623 mg, 9.9 mmole) was added, and stirring was continued for 2.5 hours. The reaction mixture was poured into sodium bicarbonate solution, and the resulting solution was extracted with ethyl acetate. The ethyl acetate extract was washed with saturated sodium chloride

solution, dried over sodium sulfate, filtered, and evaporated to give a glass. The glass was purified by silica-gel flash chromatography, eluting with a gradient of 1 L of dichloromethane to 1 L of methanol/dichloromethane (1:4) to give 1.71 g (64% yield) of 20-DH-20-O-[p-(hexahydroazepin-1-ylmethyl)phenyl]-desmycosin.

Examples 30—38

20-DH-DO-20-(Phenylthio)tylosin was prepared, using a procedure like that of Example 3, and 20-DH-DO-20-(phenythio)desmycosin was prepared using a procedure like that of Example 4.

The following tylosin derivatives were prepared using a procedure like that of Example 7:

20-DH-20-O-(p-Nitrophenyl)tylosin
20-DH-20-O-(p-Benzoylphenyl)tylosin
20-DH-20-O-(p-Methoxyphenyl)tylosin
20-DH-20-O-(3,5-Dichlorophenyl)tylosin
20-DH-20-O-[m-(N,N-Dimethylamino)phenyl]tylosin
20-DH-20-O-(p-Formylphenyl)tylosin
20-DH-20-O-(p-Phenylphenyl)tylosin

Examples 39—50

The following derivatives were prepared by procedures analogous to those of the preceding preparations and examples:

20-DH-20-O-Acetyldesmycosin
20-DH-20-O-(p-Nitrophenyl)desmycosin
20-DH-20-O-(p-Methoxyphenyl)desmycosin
20-DH-20-O-(p-Benzoylphenyl)desmycosin
20-DH-20-O-[p-(Ethoxycarbonyl)phenyl]desmycosin
20-DH-20-O-[m-(N,N-Dimethylamino)phenyl]desmycosin
20-DH-20-O-(3,5-Dichlorophenyl)desmycosin
20-DH-20-O-(p-Phenylphenyl)desmycosin
20-DH-20-O-(*p*-Phenoxyphenyl)desmycosin
20-DH-20-O-(*m*-Phenoxyphenyl)desmycosin
20-DH-20-O-Phenylactenocin

Examples 51—54

The following compounds can be prepared from the corresponding 2'-O-acyl compounds, using a procedure like that of Example 15:

20-DH-DO-20-O-N-Phthalimido-2',4'-di-O-acetyldesmycosin,
20-DH-20-O-Phenyl-2',4'-di-O-propionyldesmycosin,
20-DH-20-O-(*p*-Nitrophenyl)-2',4'-di-O-propionyldesmycosin, and
20-DH-20-O-phenyl-2'-O-acetyl-4'-O-propionyldesmycosin.

Examples 55—71

The following derivatives can be prepared by procedures analogous to those of the preceding examples:

20-DH-DO-20-[(1-Methyltetrazol-5-yl)thio]DOMM
20-DH-DO-20-[(1-Methyltetrazol-5-yl)thio]DOML
20-DH-DO-20-(Chloro)macrocin
20-DH-DO-20-(Chloro)lactenocin
20-DH-DO-20-(Phenylthio)macrocin
20-DH-DO-20-(Phenylthio)lactenocin
20-DH-20-O-(p-Nitrophenyl)macrocin
20-DH-20-O-(p-Nitrophenyl)lactenocin
20-DH-20-O-(p-Methoxyphenyl)DOMM
20-DH-20-O-(p-Methoxyphenyl)DOML
20-DH-20-O-(Phenyl)DOMM
20-DH-20-O-(Phenyl)DOML
20-DH-DO-20-(Azido)macrocin
20-DH-DO-20-(Azido)lactenocin
20-DH-DO-20-N-Phthalimido-DOMM
20-DH-DO-20-N-Phthalimido-DOML

Tables XV-XVIII which follow summarize certain physical data on illustrative compounds of this invention.

TABLE XV

Physical Characteristics of C—20-Modified Derivatives of Tylosin

| 20-Substituent | Compound[a] No. | FDMS Parent Ion | UV λmax (ε)[b] | NMR[c] δ | IR[d] $cm^{-1}$ | TLC[e] $R_f$ |
|---|---|---|---|---|---|---|
| 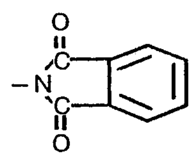 | 1 | 1047 ($m^+$+1) | 220 (42,500)<br>282 (21,500) | 7.94 (broad s, 2H)<br>7.76 (broad s, 2H) | | |
| —$N_3$ | 2 | 943 ($m^+$+1) | 282 (25,300) | 5.68 (broad s, 1H) | 2096 | |
| —OPh | 4 | 993 ($m^+$) | 278 (25,000)<br>220 (13,500) | 6.84—7.0 (m, 2H)<br>7.20—7.40 (m, 4H) | | |
| 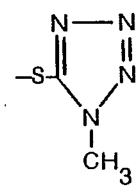 | 5 | 1016 ($m^+$+1) | 282 (22,000)<br>204 (14,500)<br>258 shoulder | 3.98 (s) | | |
| —SPh | 6 | 1010 ($m^+$+1) | (17,500)<br>281 (23,500) | 7.06—7.50 (m, 6H) | | |
| —Cl | 7 | 935 ($m^+$) | 282 (23,300) | | 0.52 | |

TABLE XV continued

| 20-Substituent | Compound[a] No. | FDMS Parent Ion | UV λmax (ε)[b] | NMR[c] δ | IR[d] $cm^{-1}$ | TLC[e] $R_f$ |
|---|---|---|---|---|---|---|
| | | | 282 (23,000) | 4.16—4.64 [2 doublets] | | |
| HN—$CO_2Et$<br>\|<br>—N—$CO_2Et$ | 9 | 1076 ($m^+$+1) | 286 (21,750) | 8.06 (s, 1H) | | 0.57 |
| —OOC—$CH_2OPh$ | 11 | 1052 ($m^+$+1) | 220 (19,500)<br>280 (22,500) | 4.68 (d, 2H)<br>6.9—7.08 (m, 2H)<br>7.2—7.4 (m, 4H) | | |
| —O—(p-$NO_2Ph$) | 12 | 1039 ($m^+$+1) | 225 (11,400)<br>287 (30,350) | 6.94 (d, 2H)<br>8.00 (d, 2H) | | |
| —O—(p-BzPh) | 13 | 1098 ($m^+$+1) | 225 (shoulder<br>(13,500)<br>285 (37,000) | 6.8—7.0 (d, 2H)<br>7.26—7.62 (m, 4H)<br>7.66—7.90 (m, 4H) | | |
| —O—(p-MeOPh) | 14 | 1024 ($m^+$+1) | 225 (11,920)<br>284 (22,500) | 3.74 (s, 3H) | | |
| —O—(3,5-diClPh) | 15 | 1062 ($m^+$) | 203 (48,000)<br>207 (46,500)<br>224 shoulder<br>(12,100)<br>228 shoulder<br>(22,100)<br>283 (22,200) | 6.8 (s, 2H)<br>6.92 (s, 1H) | | |
| —O—[m-($NMe_2$)Ph] | 16 | 1037 ($m^+$+1) | 212 (28,750)<br>260 shoulder<br>(18,000)<br>283 (24,300) | 2.94 (s, 6H) | | |

TABLE XV continued

| 20-Substituent | Compound[a] No. | FDMS Parent Ion | UV $\lambda max$ ($\varepsilon$)[b] | NMR[c] $\delta$ | IR[d] $cm^{-1}$ | TLC[e] $R_f$ |
|---|---|---|---|---|---|---|
| —O—(p-CHOPh) | 17 | 1022 ($m^+$+1) | 220 (16,500)<br>282 (41,500) | 9.88 (s, 1H)<br>6.98 (d, 2H)<br>7.80 (d, 2H) | | |
| —O—(p-PhPh) | 18 | 1069 ($m^+$) | 271 (31,200) | 9.6 (d, 2H) | | |

[a]Compound numbers from Table I
[b]In ethanol
[c]360 or 270 MHz, run in $CDCl_3$ (TMS)
[d]Run in $CHCl_3$
[e]Silica gel adsorbent; solvent system-$CH_2Cl_2$:MeOH:$NH_4OH$ (90:10:2)

TABLE XVI

Physical Characteristics of C-20-Modified Derivatives of Desmycosin

| 20-Substituent | Compound[a] No. | FDMS Parent Ion | UV λmax (ε)[b] | NMR[c] δ | IR[d] $cm^{-1}$ | TLC[e] $R_f$ |
|---|---|---|---|---|---|---|
| —Cl | 21 | 791 ($m^+$) | 282 (22,700) | | | 0.43 |
| —SPh | 23 | 865 ($m^+$) | 203 (15,700)<br>260 shoulder (17,800)<br>281 (23,300) | 7.1—7.44 (m, 6H) | | |
| 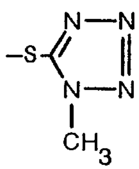 | 24 | 872 ($m^+$+1) | 282 (22,000) | 3.98 (s, 3H) | | |
| —OOC—$CH_2$OPh | 26 | 908 ($m^+$+1) | 218 shoulder (9,000)<br>282 (22,000) | 4.68 (d, 2H)<br>6.9—7.1 (m, 2H)<br>7.2—7.4 (m, 4H) | | |
| —OAc | 29 | 815 ($m^+$) | 282 (22,100) | 2.09 (s, 3H) | | |
| —OPh | 33 | 849 ($m^+$) | 220 (14,000)<br>278 (26,000) | 6.84—7.0 (m, 2H)<br>7.18—7.40 (m, 4H) | | |
| —O—(p-$NO_2$Ph) | 34 | 895 ($m^+$+1) | 227 (8,400)<br>285 (27,400) | 6.94 (d, 2H)<br>8.0 (d, 2H) | | |
| —O—(p-MeOPh) | 35 | 879 ($m^+$) | 225 (12,750)<br>283 (22,900) | 3.80 (s, 3H) | | |
| —O—(p-CHOPh) | 36 | 877 ($m^+$) | 220 (14,300)<br>284 (38,500) | 9.88 (s, 1H)<br>6.99 (d, 2H)<br>7.83 (d, 2H) | | |
| —O—(p-BzPh) | 37 | 954 ($m^+$+1) | 225 shoulder (12,300)<br>285 (34,500) | 6.95 (d, 2H)<br>7.70—7.94 (m, 4H)<br>7.42—7.66 (m, 3H) | | |

TABLE XVI continued

| 20-Substituent | Compound[a] No. | FDMS Parent Ion | UV λmax (ε)[b] | NMR[c] δ | IR[d] $cm^{-1}$ | TLC[e] $R_f$ |
|---|---|---|---|---|---|---|
| —O—(p-Et$CO_2$Ph) | 38 | 922 ($m^+$+1) | 207 shoulder (16,200)<br>265 (28,300) | 6.90 (d, 2H)<br>7.98 (d, 2H) | | |
| —O—[m-($NMe_2$)Ph] | 39 | 892 ($m^+$) | 210 (27,600)<br>257 shoulder (16,200)<br>282 (22,500) | 2.90 (s, 6H) | | |
| —O—(3,5-diClPh) | 40 | 917;919 ($m^+$) | 222 shoulder (9,800)<br>282 (21,000) | 6.80 (s, 2H)<br>6.94 (s, 1H) | | |
| —O—(p-PhPh) | 41 | 925 ($m^+$) | 272 (33,800) | 6.9—7.04 (d, 2H) | | |
| —O—[p-[$(CH_2)_6$N—$CH_2$—]Ph] | 42 | 961 ($m^+$+1) | 226 (14,200)<br>281 (22,200) | 6.83 (d, 2H)<br>7.14—7.40 (d, 2H over d, 4H) | | |
| 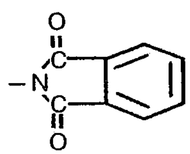 | 43 | 903 ($m^+$+1) | 219 (41,700)<br>241 (12,500)<br>282 (21,000) | 7.78 (broad s, 2H)<br>7.95 (broad s, 2H) | | |
| —$N_3$ | 44 | 798 ($m^+$) | 282 (24,000) | | 2097 | |
| HN—$CO_2$Et<br>\|<br>—N—$CO_2$Et | 45 | 931 ($m^+$) | 286 (22,000) | 8.05 (s, 1H) | | |
| —O—[p—(PhO)Ph] | 47 | 942 ($m^+$+1) | 227 (17,700)<br>281 (22,800) | 6.8—7.1 (m, 6H)<br>7.2—7.4 (m, 4H) | | |

TABLE XVI continued

| 20-Substituent | Compound[a] No. | FDMS Parent Ion | UV λmax (ε)[b] | NMR[c] δ | IR[d] $cm^{-1}$ | TLC[e] $R_f$ |
|---|---|---|---|---|---|---|
| —O—[m—(PhO)Ph] | 48 | 942 ($m^+$+1) | 220 shoulder (22,500) 281 (22,300) | 6.5—6.7 (m, 2H) 7.0—7.4 (m, 8H) | | |

[a]Compound numbers from Table II
[b]In ethanol
[c]360 or 270 MHz, run in $CDCl_3$ (TMS)
[d]Run in $CHCl_3$
[e]Silica gel adsorbent; solvent system—$CH_2Cl_2$:MeOH:$NH_4OH$ (90:10:2)

TABLE XVII
Physical Characteristics of C-20-Modified Derivatives of Macrocin

| 20-Substituent | Compound[a] No. | FDMS Parent Ion | UV $\lambda max$ ($\varepsilon$)[b] | NMR[c] $\delta$ | IR[d] $cm^{-1}$ | TLC[e] $R_f$ |
|---|---|---|---|---|---|---|
| —OPh | 53 | 980 ($m^+$+1) | 220 (18,750)<br>280 (33,500) | 6.9 (t, 3H)<br>7.25 (m, 7H) | | |

[a]Compound numbers from Table III
[b]In methanol unless otherwise indicated
[c]360 or 270 MHz, run in $CDCl_3$ (TMS)
[d]Run in $CHCl_3$
[e]Silica gel adsorbent; solvent system—$CH_2Cl_2$:MeOH:$NH_4OH$ (90:10:0.5)
[f]Run in ethanol

TABLE XVIII
Physical Characteristics of C-20-Modified Derivatives of Lactenocin

| 20-Substituent | Compound[a] No. | FDMS Parent Ion | UV λmax (ε)[b] | NMR[c] δ | TLC[d] $R_f$ |
|---|---|---|---|---|---|
| —OPh | 58 | 836 ($m^{+}$+1) | 220 (18,500)<br>280 (23,000) | | 0.50 |

[a]Compound numbers from Table IV
[b]In methanol
[c]360 or 270 MHz, run in $CDCl_3$ (TMS)
[d]Silica gel adsorbent; solvent system—$CH_2Cl_2$:MeOH:$NH_4OH$ (90:10:0.5)

Example 72

Injectable Formulations

A) A formula I base is added to propylene glycol. Water and benzyl alcohol are added so that the solution contains 50% (by volume) propylene glycol, 4% (by volume) benzyl alcohol, and 200 mg/ml of a formula I base.

B) A solution is prepared as described in Section A except that the solution contains 50 mg/ml of a formula I base.

C) A solution is prepared as described in Section A except that the solution contains 350 mg/ml of a formula I base.

D) A solution is prepared as described in Section A except that the solution contains 500 mg/ml of a formula I tartrate.

E) A suspension is prepared by adding a finely ground formula I compound to carboxymethyl cellulose with thorough mixing so that the suspension contains 200 mg of the formula I base per ml of suspension.

Example 73

Chick Ration for Control of *Mycoplasma*

A balanced, high-energy ration adapted to feed chicks for rapid weight gain is prepared by the following recipe:

| Ingredient: | % | Kg | (lbs) |
|---|---|---|---|
| Ground yellow corn | 50 | 454 | (1,000) |
| Soybean meal, solvent-extracted dehulled, finely ground, 50 percent protein | 31.09 | 282 | (621.8) |
| Animal fat (beef tallow) | 6.5 | 59 | (130) |
| Dried fish meal, with solubles (60% protein) | 5.0 | 45.4 | (100) |
| Distillers' solubles from corn | 4.0 | 36.3 | (80) |
| Dicalcium phosphate, feed grade | 1.8 | 16.3 | (36) |
| Calcium carbonate | 0.8 | 7.3 | (16) |
| Vitamin premix (representing vitamins A, D, E, K, and $B_{12}$, choline, niacin, pantothenic acid, riboflavin, biotin, with glucose bulking agent) | 0.5 | 4.5 | (10) |
| Trace mineral premix (representing $MnSO_4$, ZnO, KI, $FeSO_4$, $CaCO_3$) | 0.2 | 1.8 | (4) |
| 2-Amino-4-hydroxybutyric acid (hydroxy analog of methionine) | 0.1 | 0.9 | (2) |
| Formula I compound | 0.01 | 0.09 | (0.2) |

These substances are mixed in accordance with standard feed-mixing techniques. Chicks fed such a ration, with water *ad libitum,* are protected against exposure to *Mycoplasma* infections.

**Claims**

1. A macrolide of the formula (I)

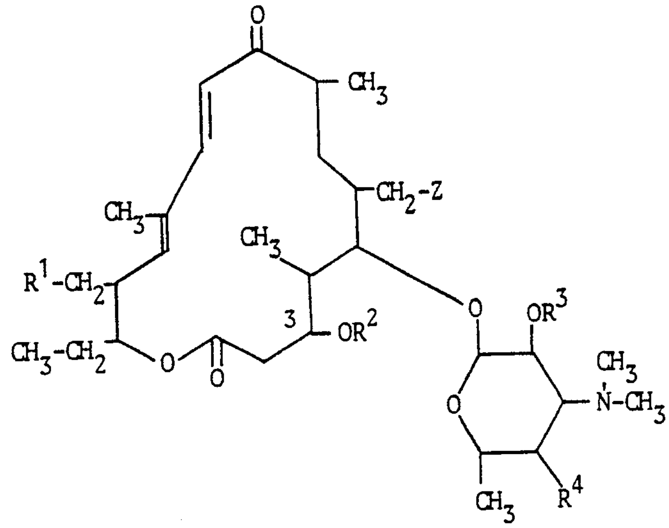

(I)

wherein

$R^1$ is

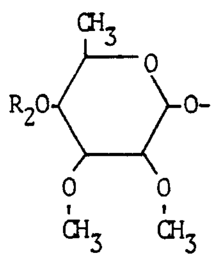 ; 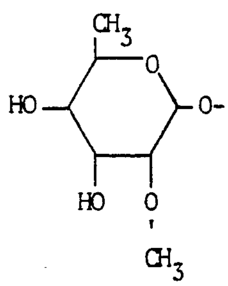 or 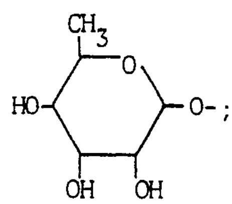

$R^2$ is hydrogen or a hydroxyl protecting group;

$R^3$ is hydrogen, optionally substituted $C_1$—$C_5$-alkanoyl optionally substituted benzoyl, optionally substituted phenylacetyl or optionally substituted phenylpropionyl;

$R^4$ is hydrogen, iodo, hydroxy, optionally substituted $C_1$—$C_5$-alkanoyloxy, optionally substituted benzoyloxy, optionally substituted phenylacetoxy or optionally substituted phenoxyacetoxy or

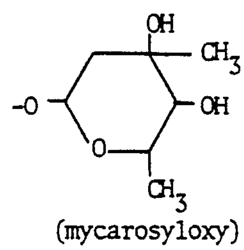

(mycarosyloxy) ;

Z is a group —$CH_2R$, where R is chloro, bromo, azido, or

R is a group —O—$R^{5'}$ where $R^{5'}$ is phenyl, derivatized phenyl, or naphthyl or

R is a group

$$-O-\overset{\overset{\displaystyle O}{\|}}{C}R^6$$

where $R^6$ is optionally substituted $C_1$—$C_4$ alkyl, phenyl, derivatized phenyl, optionally substituted phenoxy, or optionally substituted phenylthio, or

R is a group —$OSO_2R^7$ where $R^7$ is methyl, trifluoromethyl, or optionally substituted phenyl, or

R is a group —$SR^9$ where $R^9$ is optionally substituted $C_1$—$C_4$ alkyl, cyclohexyl, phenyl, derivatized phenyl, optionally substituted benzyl, optionally substituted phenylacetyl, or

an optionally substituted heteroaryl group selected from imidazolyl, pyrazolyl, pyridinyl, pyrimidinyl, pyrazinyl, pyridazinyl, triazinyl, triazolyl, tetrazolyl, oxazolyl, isoxazolyl, oxadiazolyl, thiazolyl, isothiazolyl, thiadiazolyl, thienyl and furanyl, or

R is a group

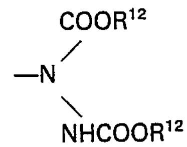

where $R^{12}$ is methyl, or ethyl, or

R is phthalimido or succinimido, or an acid addition salt thereof;
provided that when Z is $-CH_2Br$ then $R^1$ is

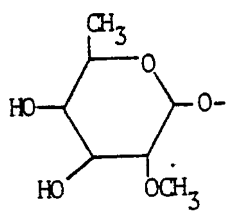

or

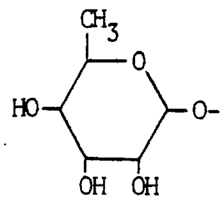

;

and provided that if $R^4$ is hydrogen or iodo, then $R^1$ is

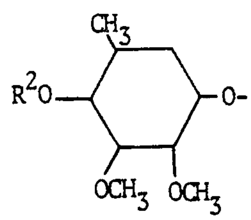

and provided that $R^2$ is hydrogen unless $R^4$ is hydrogen or iodo.

2. A process for preparing a macrolide of formula (I) or a pharmaceutically-acceptable salt thereof, which comprises reacting a starting macrolide of formula (II)

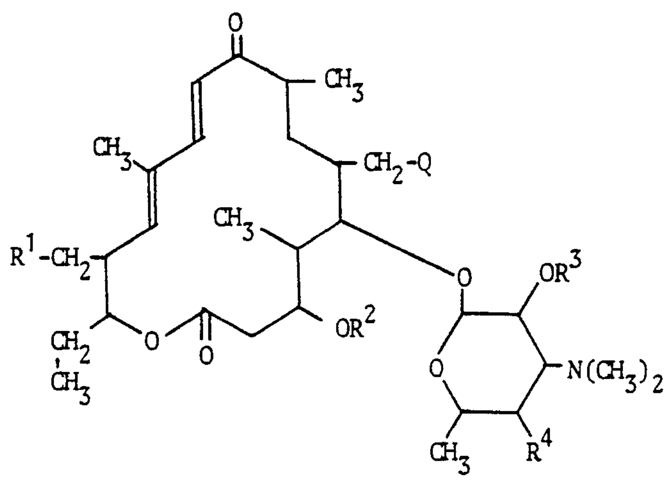

(II)

wherein $R^1$, $R^2$, $R^3$, and $R^4$ are as defined in formula (I), and where Q is $-CH_2OH$, with triphenylphosphine and a halogenating agent to provide a macrolide of formula (I) wherein Z is $-CH_2Cl$ or $-CH_2Br$, or with triphenyl phosphine and diethylazodicarboxylate or dimethylazodicarboxylate to provide a macrolide or formula (I) where Z is

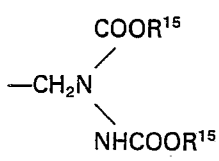

and $R^{15}$ is methyl or ethyl, or with triphenylphosphine, and diethylazodicarboxylate or dimethylazodicarboxylate, and a reagent selected from

(a) an azide transfer reagent to provide a macrolide of formula (I) wherein Z is $—CH_2N_3$

(b) phthalimide or succinimide to provide macrolide of formula (I) wherein Z is $—CH_2—$ phthalimido or $—CH_2—$succinimido

(c) a phenol of formula $HOR^5$, where $R^5$ is phenyl or derivatized phenyl, to provide a macrolide of formula (I) wherein Z is $—CH_2OR^5$,

(d) a carboxylic acid of formula $R^6COOH$, where $R^6$ is as defined in formula (I), to provide a macrolide of formula (I) wherein Z is $R^6COOCH_2—$,

(e) an alkyl halide or polyhalide to provide a macrolide of formula (I) wherein Z is $—CH_2Br$ or $—CH_2Cl$,

(f) a sulfonic acid of the formula $R^7SO_2OH$, where $R^7$ is as defined in formula (I) to provide a macrolide of formula (I) wherein Z is $—CH_2OSO_2R^7$,

(g) a mercaptan of the formula $HSR^9$, where $R^9$ is as defined in formula (I) to provide a macrolide of formula (I) wherein Z is $—CH_2SR^9$.

or hydrolyzing a macrolide of formula (I) wherein $R^4$ is mycarosyloxy in acid solution at a pH below 4 to provide a corresponding macrolide of formula (I) wherein $R^4$ is hydroxy;

or reacting a macrolide of formula (I) wherein $R^4$ is a sulfonate with a source of iodide ion to provide a macrolide of formula (I) wherein $R^4$ is iodo;

or reductively deiodinating a macrolide of formula (I) wherein $R^4$ is iodo to provide a macrolide of formula (I) wherein $R^4$ is hydrogen; and

optionally esterifying or salifying the macrolide provided by any of the foregoing steps.

3. A macrolide of formula (I) or a pharmaceutically acceptable salt thereof as claimed in claim 1 for use as an antibiotic in the chemotherapy of warm-blooded animals.

4. A feed premix comprising as an active ingredient a macrolide of formula (I), or a pharmaceutically acceptable salt thereof, as claimed in claim 1.

5. A veterinary formulation which comprises as an active ingredient a macrolide of formula (I), or a pharmaceutically-acceptable salt thereof, as claimed inclaim 1 associated with one or more physiologically-acceptable carriers or vehicles therefor.

**Patentansprüche**

1. Macrolid der Formel (I)

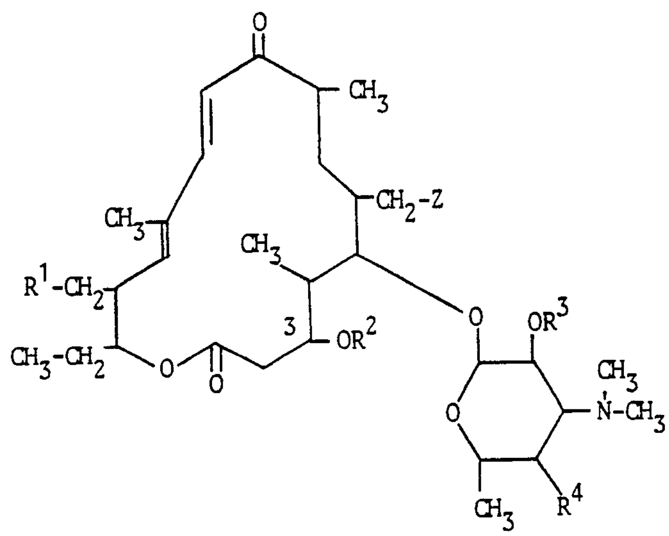

(I)

worin $R^1$ für

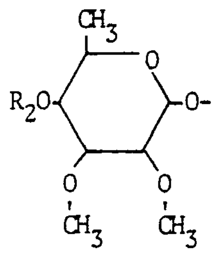

;

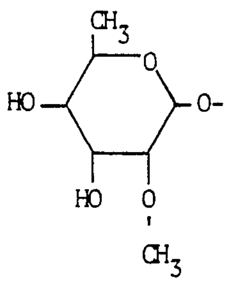

oder

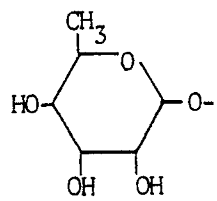

steht,

$R^2$ Wasserstoff oder eine Hydroxlschutzgruppe ist,

$R^3$ Wasserstoff, gegebenenfalls substituiertes $C_1$—$C_5$-Alkanoyl, gegebenenfalls substituiertes Benzoyl, gegebenenfalls substituiertes Phenylacetyl oder gegebenenfalls substituiertes Phenylpropionyl ist,

$R^4$ Wasserstoff, Iod, Hydroxy, gegebenenfalls substituiertes $C_1$—$C_5$-Alkanoyloxy, gegebenenfalls substituiertes Benzoyloxy, gegebenenfalls substituiertes Phenylacetoxy oder gegebenenfalls substituiertes Phenoxyacetoxy oder der Rest

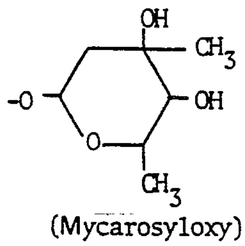

(Mycarosyloxy)

ist,

Z eine Gruppe der Formel —$CH_2R$ bedeutet, worin R Chlor, Brom oder Azido darstellt oder R eine Gruppe der Formel —O—$R^{5'}$ ist, worin $R^{5'}$ Phenyl derivatisiertes Phenyl oder Naphthyl bedeutet,

oder R eine Gruppe der Formel

$$-O-\overset{\overset{\large O}{\|}}{C}R^6$$

ist,

worin $R^6$ gegebenenfalls substituiertes $C_1$—$C_4$-Alkyl, Phenyl, derivatisiertes Phenyl, gegebenenfalls substituiertes Phenoxy oder gegebenenfalls substituiertes Phenylthio bedeutet, oder R eine Gruppe der Formel —$OSO_2R^7$ ist, worin $R^7$ Methyl, Trifluoromethyl oder gegebenenfalls substituiertes Phenyl bedeutet,

oder R eine Gruppe der Formel —$SR^9$ ist, worin $R^9$ gegebenenfalls substituiertes $C_1$—$C_4$-Alkyl, Cyclohexyl, Phenyl, derivatisiertes Phenyl, gegebenenfalls substituiertes Benzyl, gegebenenfalls substituiertes Phenylacetyl oder eine gegebenenfalls substituiertes Heteroarylgruppe bedeutet, die ausgewählt ist aus Imidazolyl, Pyrazolyl, Pyridinyl, Pyrimidinyl, Pyrazinyl, Pyridazinyl, Triazinyl, Triazolyl, Tetrazolyl, Oxazolyl, Isoxazolyl, Oxadiazolyl, Thiazolyl, Isothiazolyl, Thiadiazolyl, Thienyl oder Furanyl, oder R eine Gruppe der Formel

$$-N\begin{matrix} \diagup COOR^{12} \\ \diagdown NHCOOR^{12} \end{matrix}$$

ist, worin $R^{12}$ Methyl oder Ethyl bedeutet, oder R Phthalimido oder Succinimido ist, oder ein Säureadditionssalz hiervon, wobei jedoch, falls Z für —$CH_2Br$ steht, der Substituent $R^1$

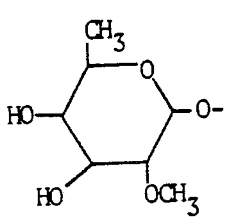 oder 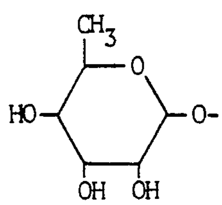

bedeutet,

und, falls $R^4$ Wasserstoff oder Iod ist, der Substituent $R^1$

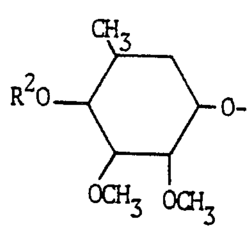

bedeutet, und $R^2$ Wasserstoff ist, falls $R^4$ nicht für Wasserstoff oder Iod steht.

2. Verfahren zur Herstellung eine Macrolids der Formel (I) oder eines pharmazeutisch annehmbaren Salzes hiervon, dadurch gekennzeichnet, daß man ein Ausgangsmacrolid der Formel (II)

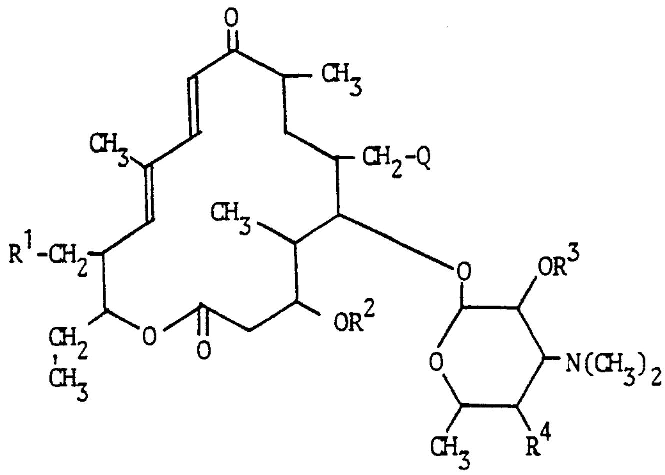

(II)

worin $R^1$, $R^2$, $R^3$ und $R^4$ wie bei der Formel (I) definiert sind und worin Q für $-CH_2OH$ steht, mit Triphenylphosphin und einem Halogeniergungsmittel umsetzt und so ein Macrolid der Formel (I) bildet, worin Z für $-CH_2Cl$ oder $-CH_2Br$ steht, oder mit Triphenylphosphin und Diethylazodicarboxylat oder Dimethylazodicarboxylat umsetzt und so ein Macrolid der Formel (I) bildet, worin Z für

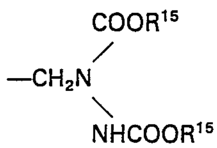

steht, wobei $R^{15}$ Methyl oder Ethyl ist, oder mit Triphenylphosphin und Diethylazodicarboxylat oder Dimethylazodicarboxylat und einem Reagens umsetzt, das ausgewählt ist aus

(a) einem Azidtransferreagens, wodurch ein Macrolid der Formel (I) gebildet wird, worin Z für $-CH_2N_3$ steht,

(b) Phthalimid oder Succinimid, wodurch ein Macrolid der Formel (I) gebildet wird, worin Z für $-CH_2-$Phthalimido oder $-CH_2$-Succinimido steht,

(c) einem Phenol der Formel $HOR^5$, worin $R^5$ Phenyl oder derivatisiertes Phenyl ist, wodurch eine Macrolid der Formel (I) gebildet wird, worin Z für $-CH_2OR^5$ steht,

(d) einer Carbonsäure der Formel $R^6COOH$, worin $R^6$ wie bei der Formel (I) definiert ist, wodurch ein Macrolid der Formel (I) gebildet wird, worin Z für $R^6COOCH_2$-steht.

(e) einem Alkylhalogenid oder Alkylpolyhalogenid, wodurch ein Macrolid der Formel (I) gebildet wird, worin Z für $-CH_2Br$ oder $-CH_2Cl$ steht,

(f) einer Sulfonsäure der Formel $R^7SO_2OH$, worin $R^7$ wie bei der Formel (I) definiert ist, wodurch ein Macrolid der Formel (I) gebildet wird, worin Z für $-CH_2OSO_2R^7$ steht, oder

(g) einem Mercaptan der Formel $HSR^9$, worin $R^9$ wie bei der Formel (I) definiert ist, wodurch ein Macrolid der Formel (I) gebildet wird, worin Z für $-CH_2SR^9$ steht,

oder ein Macrolid der Formel (I), worin $R^4$ Mycarosyloxy ist, in einer Säurelösung bei einem pH-Wert von unter 4 hydrolisiert, wodurch das entsprechende Macrolid der Formel (I) gebildet wird, worin $R^4$ Hydroxy ist, oder ein Macrolid der Formel (I), worin $R^4$ ein Sulfonat ist, mit einer Quelle für Iodidionen umsetzt, wodurch ein Macrolid der Formel (I) gebildet wird, worin $R^4$ Iod ist, oder ein Macrolid der Formel (I), worin $R^4$ Iod ist, reduktiv deiodiert, wodurch ein Macrolid der Formel (I) gebildet wird, worin $R^4$ Wasserstoff ist, und das nach irgendeiner der vorherigen Stufen erhaltene Macrolid gegenbenenfalls verestert oder in ein Salz überführt.

3. Verwendung eines Macrolids der Formel (I) oder eines pharmazeutisch annehmbaren Salzes hiervon nach Anspruch 1 als Antibioticum bei der chemotherapeutischen Behandlung warmblütiger Tiere.

4. Futtervormischung, dadurch gekennzeichnet, daß sie eine Verbindung der Formel (I) oder eine pharmazeutisch annehmbares Salz hiervon nach Anspruch 1 als Wirkstoff enthält.

5. Veterinärformulierung, dadurch gekennzeichnet, daß sie ein Macrolid der Formel (I) oder ein pharmazeutisch annehmbares Salz hiervon nach Anspruch 1 als Wirkstoff in Verbindung mit einem oder mehreren physiologisch annehmbaren Trägern oder Schleppmitteln hierfür enthält.

## Revendications

1. Macrolide répondant à la formule (I)

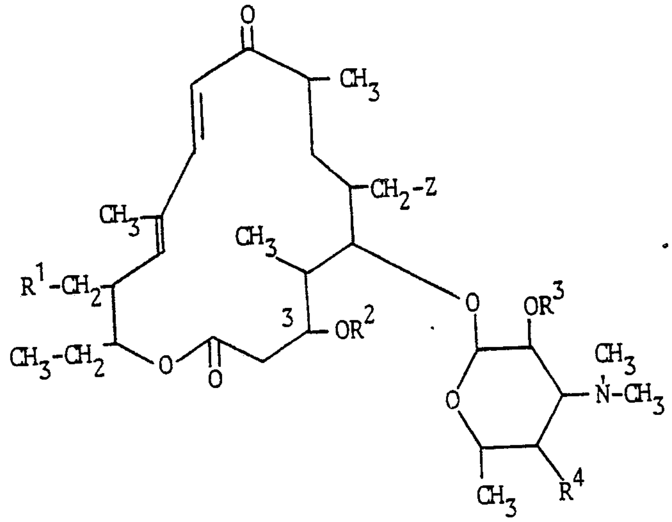

(I)

dans laquelle

$R^1$ représente

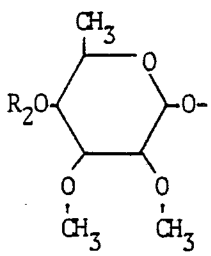

;

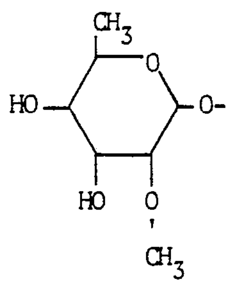

ou

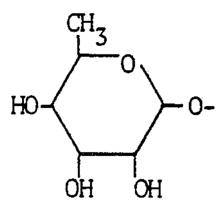

$R^2$ représente un atome d'hydrogène ou un groupe protecteur du groupe hydroxyle;

$R^3$ représente un atome d'hydrogène, un groupe alcanoyle en $C_1$—$C_5$ facultativement substitué un groupe benzoyle facultativement substitué, un groupe phénylacétyle facultativement substitué ou un groupe phényl-propionyle facultativement substitué;

$R^4$ représente un atome d'hydrogène, un atome d'iode, un groupe hydroxyle, un groupe alcanoyl (en $C_1$—$C_5$)oxy facultativement substitué, un groupe benzoyloxy facultativement substitué, un groupe phénylacétoxy facultativement substitué ou un groupe phénoxyacétoxy facultativement substitué ou encoure le groupe

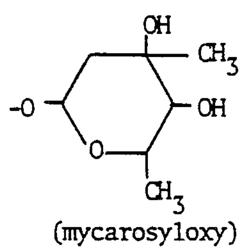

(mycarosyloxy) ;

Z représente un groupe —$CH_2R$, où R représente un atome de chlore, un atome de brome ou un groupe azido, ou

R représente un groupe —O—$R^{5'}$ où $R^{5'}$ représente un groupe phényle, un groupe phényle à dérivation ou un groupe naphtyle; ou

R représente un groupe

$$—O—\overset{\overset{O}{\|}}{C}R^6$$

où $R^6$ représente un groupe alkyle ne $C_1$—$C_4$ facultativement substitué, un groupe phényle, un groupe phényle à dérivation, un groupe phénoxy facultativement substitué ou un groupe phénylthio facultativement substitué; ou

R représente un groupe —$OSO_2R^7$ où $R^7$ représente un groupe méthyle, un groupe trifluorométhyle ou un groupe phényle facultativement substitué; ou

R représente un groupe —$SR^9$ où $R^9$ représente un groupe alkyle en $C_1$—$C_4$ facultativement substitué, un groupe cyclohexyle, un groupe phényle, un groupe phényle à dérivation, un groupe benzyle facultativement substitué, un groupe phénylacétyle facultativement substitué ou un groupe hétéro-aryle facultativement substitué choisi parmi les groupes imidazolyle, pyrazolyle, pyridinyle, pyrimidinyle, pyrazinyle, pyridazinyle, triazinyle, triazolyle, tétrazolyle, oxazolyle, isoxazolyle, oxadiazolyle, thiazolyle, isothiazolyle, thiadiazolyle, thiényle et furanyle; ou

R représente un groupe

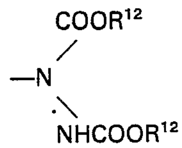

où $R^{12}$ représente un groupe méthyle ou un groupe éthyle; ou

R représente un groupe phtalimido ou succinimido;

ou un sel d'addition d'acide de ce macrolide; avec cette réserve que, si Z représente —$CH_2Br$, $R^1$ représente

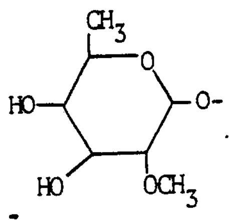 ou 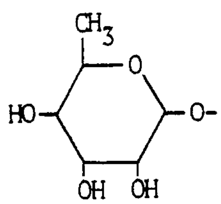

et que si $R^4$ représente un atome d'hydrogène ou d'iode, $R^1$ représente

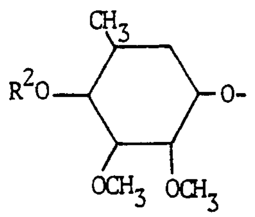

et que $R^2$ représente un atome d'hydrogène à moins que $R^4$ ne représente un atome d'hydrogène ou un atome d'iode.

2. Procédé de préparation d'un macrolide répondant à la formule (I) ou d'un de ses sels pharmaceutiquement acceptables, caractérisé en ce qu'il consisté à faire réagir un macrolide de départ répondant à la formule (II):

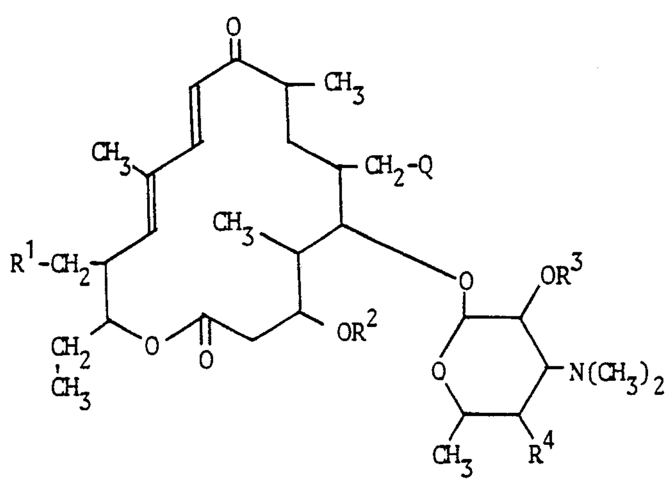

(II)

dans laquelle $R^1$, $R^2$, $R^3$ et $R^4$ ont les significations définies en formule (I) et où Q représente —$CH_2OH$, avec de la triphénylphosphine et un agent d'halogénation pour obtenir un macrolide de formule (I) dans laquelle Z représente —$CH_2Cl$ ou —$CH_2Br$ ou avec de la triphénylphosphine et un azodicarboxylate de diéthyle ou de diméthyle pour obtenir un macrolide de formule (I) dans laquelle Z représente

$$
\begin{array}{l}
\qquad\quad COOR^{15} \\
\qquad\ / \\
-CH_2N \\
\qquad\ \backslash \\
\qquad\quad NHCOOR^{15}
\end{array}
$$

et $R^{15}$ représente un groupe méthyle ou éthyle;

ou avec de la triphénylphosphine et un azodicarboxylate de diéthyle ou de diméthyle, ainsi qu'avec un réactif choisi parmi

(a) un réactif de transfert d'azide pour obtenir un macrolide de formule (I) dans laquelle Z représente —$CH_2N_3$;

(b) un phtalamide ou un succinimide pour obtenir un macrolide de formule (I) dans laquelle Z représente —$CH_2$-phtalimido ou —$CH_2$-succinimido;

(c) un phénol de formule $HOR^5$, où $R^5$ représente un groupe phényle ou un groupe phényle à dérivation, pour obtenir un macrolide de formule (I) dans laquelle Z représente —$CH_2OR^5$;

(d) un acide carboxylique de formule $R^6COOH$, où $R^6$ a la signification définie en formule (I), pour obtenir un macrolide de formule (I) dans laquelle Z représente $R^6COOCH_2$—;

(e) un halogénure ou un polyhalogénure d'alkyle pour obtenir un macrolide de formule (I) dans laquelle Z représente —$CH_2Br$ ou —$CH_2Cl$;

(f) un acide sulfonique de formule $R^7SO_2OH$, où $R^7$ a la signification définie en formule (I), pour obtenir un macrolide de formule (I) dans laquelle Z représente —$CH_2OSO_2R^7$;

(g) un mercaptan de formule $HSR^9$ où $R^9$ a la signification définie en formule (I), pour obtenir un macrolide de formule (I) dans laquelle Z représente —$CH_2SR^9$;

ou hydrolyser un macrolide de formule (I) dans laquelle $R^4$ représente le groupe mycarosyloxy en solution acide à un pH inférieur à 4 pour obtenir un macrolide correspondant de formule (I) où $R^4$ représente un groupe hydroxyle;

ou faire réagir un macrolide de formule (I) dans laquelle $R^4$ représente un sulfonate, avec une source d'ions iodure pour obtenir um macrolide de formule (I) où $R^4$ représente un atome d'iode;

ou procéder à une désiodation réductrice d'un macrolide formule (I) dans laquelle $R^4$ représente un atome d'iode pour obtenir un macrolide de formule (I) dans laquelle $R^4$ représente un atome d'hydrogène;

et éventuellement estérifier ou salifier le macrolide obtenu par l'une ou l'autre des étapes précédentes.

3. Macrolide de formule (I) ou un de ses sels pharmaceutiquement acceptables selon la revendication 1, caractérisé en ce qu'on l'utilise comme antibiotique dans la chimiothérapie des animaux à sang chaud.

4. Prémélange de nourriture, caractérisé en ce q'uil comprend, comme ingrédient actif, un macrolide de formule (I) ou un des sels pharmaceutiquement acceptables selon la revendication 1.

5. Formulation vétérinaire caractérisé en ce qu'elle comprend comme ingrédient actif, un macrolide de formule (I) ou un de ses sels pharmaceutiquement acceptables, selon la revendication 1, en association avec un ou plusieurs véhicules ou supports physiologiquement acceptables pour ce macrolide ou ce sel.